# EUROPEAN PATENT SPECIFICATION

(11) **EP 1 520 039 B1**
(45) Date of publication and mention of the grant of the patent: **13.08.2008**
(21) Application number: 03718946.1
(22) Date of filing: 23.04.2003
(51) Int. Cl.: C12Q 1/68, G01N 33/50

(54) **MITOCHONDRIAL POLYMORPHISMS LINKED TO A PREDISPOSITION FOR THE DEVELOPMENT OF INAPPROPRIATE SCARRING OR FIBROSIS**
MITOCHONDRIALE POLYMORPHISMEN, DIE IN ZUSAMMENHANG MIT EINER PRÄDISPOSITION FÜR DIE ENTWICKLUNG VON UNGEEIGNETEM NARBENGEWEBE ODER FIBROSE STEHEN
POLYMORPHISMES MITOCHONDRIAUX ASSOCIES A UNE PREDISPOSITION AU DEVELOPPEMENT DE FIBROSE OU DE CICATRISATION INAPPROPRIEE

(30) Priority: 30.04.2002 GB 0209812
(43) Date of publication of application: 06.04.2005
(73) Proprietor: Renovo Limited, Manchester M13 9XX (GB)
(72) Inventor: FERGUSON, Mark William James Bank End Barn, Derbyshire SK23 7PX (GB); OLLIER, William Ernest Royce, Cheshire CW4 8JB (GB); BAYAT, Ardeshir School of Biological Sciences, Manchester M13 9PT (GB)
(74) Representative: Banford, Paul Clifford
(86) International application number: PCT/GB2003/001717
(87) International publication number: WO 2003/093506

(56) References cited:
- WO-A-80/02294
- DUCLUZEAU PIERRE-HENRI ET AL: "Depletion of mitochondrial DNA associated with infantile cholestasis and progressive liver fibrosis." JOURNAL OF HEPATOLOGY, vol. 30, no. 1, January 1999 (1999-01), pages 149-155, XP000104563 ISSN: 0168-8278
- TANAKA ATSUSHI ET AL: "Genomic analysis of differentially expressed genes in liver and biliary epithelial cells of patients with primary biliary cirrhosis." JOURNAL OF AUTOIMMUNITY, vol. 17, no. 1, August 2001 (2001-08), pages 89-98, XP002251335 ISSN: 0896-8411
- PICCI L ET AL: "SCREENING FOR CYSTIC FIBROSIS GENE MUTATIONS BY MULTIPLEX DNA AMPLIFICATION" HUMAN GENETICS, BERLIN, DE, vol. 88, no. 5, 1992, pages 552-556, XP000918185

## Description

The present invention relates to methods for the genetic testing of samples to determine the presence of polymorphisms or mutations that are linked to a genetic predisposition to develop conditions at least partially characterised by inappropriate scarring or fibrosis.

A scar is an abnormal morphological structure resulting from a previous injury or wound (e.g. an incision, excision or trauma). Scars are composed of a connective tissue which is predominately a matrix of collagen types 1 and 3 and fibronectin. The scar may consist of collagen fibres in an abnormal organisation (as seen in normal scars of the skin) or it may be, an abnormal accumulation of connective tissue (as seen in scars of the central nervous system or pathological scarring of the skin).

Scarring is a usual consequence of the healing process in most adult animal and human tissues. In the skin scars may be depressed below the surface or elevated above the surface of the skin. Hypertrophic scars are a more severe form of scarring that can arise in certain conditions or certain individuals. Hypertrophic scars are elevated above the normal surface of the skin and contain excessive collagen arranged in an abnormal pattern. A keloid is a form of pathological scarring which is not only elevated above the surface of the skin but also extends beyond the boundaries of the original injury. In a keloid there is excessive connective tissue which is organised in an abnormal fashion predominantly in whorls of collagenous tissue.

There are believed to be genetic predispositions to forming inappropriate, and particularly pathological, scarring (e.g. hypertrophic scars and keloids). For instance, Africo-Carribean and Mongoloid races are particularly prone to developing keloid scars. Furthermore a predisposition to developing hypertrophic scars and keloids may run in families.

There are numerous medical situations where scar formation represents a problem. Examples of such situations are scars of the skin where excessive scarring may be detrimental to tissue function and particularly when scar contracture occurs (for instance skin bums and wounds that impair flexibility of a joint). The reduction of scarring to the skin when cosmetic considerations are important is also highly desirable. In the skin, hypertrophic or keloid scars (particularly in Africo-Caribbean and Mongoloid races) can cause functional and cosmetic impairment and there is a need to prevent their occurrence. Scarring resulting from skin grafts in both donor sites and from the application of artificial skin can also be problematic and need to be minimised or prevented. Given the importance of scarring in such situations, it will be appreciated that there is a need to be able to test a subject to investigate whether or not they will be susceptible to developing inappropriate scarring.

As well as scars of the skin, internal scarring or fibrosis can be highly detrimental and specific examples include:
(i) Within the central nervous system, glial scarring can prevent neuronal reconnection (e.g. following neuro-surgery or penetrating injuries of the brain).
(ii) Scarring in the eye can be detrimental. In the cornea, scarring can result in abnormal opacity and lead to problems with vision or even blindness. In the retina, scarring can cause buckling or retinal detachment and consequently blindness. Scarring following wound healing in operations to relieve pressure in glaucoma (e.g. glaucoma filtration surgery) results in the failure of the surgery whereby the aqueous humour fails to drain and hence the glaucoma returns.
(iii) Scarring in the heart (e.g. following surgery or myocardial infarction) can give rise to abnormal cardiac function.
(iv) Operations involving the abdomen or pelvis, often result in adhesion between viscera. For instance, adhesions between elements of the gut and the body wall may form and cause twisting in the bowel loop leading to ischaemia, gangrene and the necessity for emergency treatment (untreated they may even be fatal). Likewise, trauma or incisions to the guts can lead to scarring and scar contracture to strictures which cause occlusion of the lumen of the guts which again can be life threatening.
(v) Scarring in the pelvis in the region of the fallopian tubes can lead to infertility.
(vi) Scarring following injury to muscles can result in abnormal contraction and hence poor muscular function.
(vii) Scarring or fibrosis following injury to tendons and ligaments can result in serious loss of function.

Related to the above is the fact that there are a number of medical conditions known as fibrotic disorders in which the development of excessive fibrotic tissue leads to pathological derangement and malfunctioning of tissue. Fibrotic disorders are
characterised by the accumulation of fibrous tissue (predominately collagens) in an abnormal fashion within the tissue. Accumulation of such fibrous tissues may result from a variety of disease processes. These diseases do not necessarily have to be caused by surgery, traumatic injury or wounding. Fibrotic disorders are usually chronic. Examples of fibrotic disorders include cirrhosis of the liver, liver fibrosis, glomerulonephritis, pulmonary fibrosis, cystic fibrosis, scleroderma, myocardial fibrosis, fibrosis following myocardial infarction, central nervous system fibrosis following a stroke or neuro-degenerative disorders (e.g. Alzheimer's Disease), proliferative vitreoretinopathy (PVR) and arthritis.

As is the case for scarring, it is believed that there is a genetic influence on the development of many fibrotic disorders or the severity thereof. In some instances, the genetic influence may be directly responsible for the development of the disorder whereas for other fibrotic disorders there may be a genetic factor that influences fibrotic development which is secondary to the primary cause of the disorder (e.g. in cystic fibrosis).

One example of a disorder that involves fibrosis, and is also believed to be influenced by genetics, is Dupuytren's disease (DD).

DD is a nodular palmar fibromatosis causing progressive and permanent contracture of the digits. DD is an irreversible, progressive disorder with a high rate of recurrence after surgical excision. It is often familial and is common in individuals of Northern European extraction. In excess of 25% of males of Celtic races over 60 years of age have evidence of DD and it is considered to be one of the most common heritable disorders of connective tissue in Caucasians.

No single gene has so far been identified in the literature as being responsible for the condition. Furthermore it is unclear whether DD is a complex oligogenic condition or a simple monogenic Mendelian disorder. Accordingly the identification of susceptible genetic loci would provide an ideal approach to unravelling the hereditary component of this common disease and would also be valuable in the subsequent development of treatment and management strategies for DD.

It will be appreciated from the above that there is a need to be able to assess individuals for susceptibility for developing conditions at least partially characterised by inappropriate scarring or fibrosis and also to assess the prognosis for an individual suffering from such a condition. One way in which this may be approached is the identification of polymorphisms or mutations in a gene that may be associated with a particular medical condition. Once identified, such polymorphisms or mutations can provide useful information to physicians who need to manage, treat or establish a susceptibility to developing a condition. One way in which the information may be used is in the development of a genetic test.

Genetic testing may be defined as the analytical testing of a patient's nucleic acid to determine if the DNA of a patient contains mutations (or polymorphisms) that either cause or increase susceptibility to a condition or are in association with the gene causing the condition and are thus potentially indicative of a predisposition to that condition.

The early detection of a predisposition to a condition presents the best opportunity for medical intervention. Early genetic identification of risk may improve the prognosis for a patient through early intervention before clinical symptoms manifest.

In cases where patients with similar symptoms are treated with variable success with the same therapeutics, genetic testing may differentiate patients with a genetic rather than developmental basis for their symptoms, thus leading to the potential need for different approaches to therapy.

It is an aim of the present invention to provide methods for genetic screening to indicate a risk or predisposition to conditions at least partially characterised by inappropriate fibrosis or scarring.

According to a first aspect of the present invention there is provided an *in vitro* method for diagnosing or detecting a predisposition to a condition at least partially characterised by inappropriate fibrosis or scarring, the method comprising examining the 16srRNA of the mitochondrial genome from a subject of interest to detect the presence of a genetic polymorphism or mutation linked to the development of the condition.

The method according to the first aspect of the invention allows an investigator to identify a subject expressing genetic polymorphisms or mutations in the 16srRNA of the mitochondrial genome to determine those subjects who have, or are more at risk of developing, a condition at least partially characterised by inappropriate fibrosis or scarring. This allows for appropriate action to be taken to prevent or lessen the likelihood of onset of the condition or to allow appropriate treatment thereof. The method is also useful for establishing a prognosis for a subject that has already been diagnosed as suffering form a particular condition.

By "polymorphism" we mean a region of a gene, or regulating elements thereof, where the nucleotide base sequence may vary between individuals. There is often a predominant genotype that represents the usual form, or wild type, of a gene with subsets of a population having a polymorphism which confers a different genotype. Certain polymorphisms can be prevalent in individuals of particular ethnic backgrounds, or from specific geographical areas. A polymorphism may not affect function of the gene; may lead to differences in the function of the gene; may produce an inactive gene product; or may modulate the production of the gene product.

By "mutation" we mean a region of a gene, or regulating elements thereof, where the nucleotide base sequence varies from the usual genotype (i.e. the wildtype). The mutation may be a base substitution, addition or deletion. A mutation may not affect function of the gene; may lead to differences in the function of the gene; may produce an inactive gene product; or may modulate the production of the gene product.

By "regulatory elements" we mean the DNA that is involved in regulating gene transcription. For instance, transcription factor binding sequences, the TATA box and in particular the PL and PH1 promoter. The PL and PH1 promoter is a predominant promoter located in the control region which includes the displacement (D)-loop and influences transcription of all mitochondrial genes.

By "mitochondrial genome" we mean the genetic material found within the mitochondria of cells of a subject being tested. This genetic material includes all genes and particularly coding sequences and regulatory elements thereof.

The mitochondrial genome is a double stranded DNA circle of 16,558 base pairs (less than 10⁻⁵ times the size of the nuclear genome) compartmentalised within each mitochondria. Mitochondrial DNA (mtDNA) is typically present in 10³-10⁴ identical copies in each cell. There are however, 10² copies in the sperm and 10⁵ in the oocyte.

Mitochondria are recognized as important and vital components of the cytoplasm of eukaryotic cells. Mitochondria are responsible for generation of energy in the form of adenosine triphosphate (ATP) through the process of oxidative phosphorylation (OXPHOS). Mitochondria also play a role in the regulation of programmed cell death, or apoptosis. The mitochondrial inner membrane contains a number of apoptotic promoting factors such as cyt c, Smac, DIABLO, AIF and caspases. Opening of the mitochondrial permeability transition pore causes swelling of the membrane and release of these apoptotic factors into the cellular cytoplasm.

By comparison to the nuclear genome, mtDNA is extremely compact with only 1kb of non-coding sequence known as the displacement loop (D-loop). The D-loop forms a triplex and is the site of origin of mitochondrial replication. There are no introns and mtDNA is not covered by protective histones like nuclear DNA. The mitochondrial genome is tethered to the inner mitochondrial membrane close to the respiratory chain.

MtDNA is comprised of 13 protein-coding genes. The complete sequence of human mtDNA is known to those skilled in the art as the Cambridge Reference Sequence (CRS) (Anderson et al., Nature 1981 Apr 9; 290(5806): 457-65). The mtDNA was recently re-sequenced and the CRS further revised (Andrews et al., Nat Genet 1999 Oct; 23(2): 147).

The sequences of mtDNA can typically vary by 50 nucleotides (0.3%) in unrelated humans. A single mtDNA sequence variant usually exists in all cells of most humans. This is called homoplasmy.

However the mutation rate in human mtDNA is 10-20 times that of nuclear DNA. This mutation rate is thought to be at least partially due to failure of proof reading mtDNA polymerase enzymes. Furthermore, the respiratory chain is a potent source of oxygen free radicals which are believed to cause mutations to occur in mtDNA.

Variations in the sequence of mtDNA can either be inherited or created *in situ* (somatic).

Mitochondrial disorders are among the most common groups of inborn errors of metabolism with an estimated incidence of 1 in 10,000 live births. Mitochondrial conditions relate to diseases in the mitochondrial oxidative phosphorylation (OXPHOS) system, the pathway leading to the production of ATP. Eighty five proteins are involved in the complex OXPHOS system encoded for by both the mitochondrial and nuclear genome hence the variety of clinical phenotypes seen in genetic mutations of the OXPHOS system.

There are four fundamental differences between Mendelian and mitochondrial genetics, which include inheritance by maternal lineage, polyplasmy, heteroplasmy and the threshold effect. The phenotype and clinical expression of a specific pathogenic mtDNA mutation is affected by the position of the mutation within the mitochondrial genome but also by the proportion of mutant to wildtype mitochondria within the cell. This is sometimes referred to as mutational load; the degree of heteroplasmy for an mtDNA mutation within the cell.

The threshold effect refers to a critical number of mutated DNAs that need to be present for a mitochondrial dysfunction to occur. The threshold found in a biochemical expression could have 60% mutations as mtDNA deletions and 95% mutations found in tRNA. The energy requirements of a specific tissue could affect the degree of tissue dysfunction.

MtDNA mutations comprise point mutations (substitutions) and rearrangements (deletions and duplications) but no splice site mutations, as there are no introns. Point mutations are commonly thought to be maternally inherited but rearrangements are often sporadic.

Only females transmit mitochondrially based traits (inherited mitochondrial mutations). A trait is transmitted directly from generation to generation. This suggests dominant inheritance but differs from classic Mendelian genetics. Both males and females are affected. Females transmit the trait to all their offspring, which does not fit with transmission of genes in the nucleus. The consensus view therefore is that under normal conditions mammalian mtDNA is inherited exclusively down the maternal line. There is a selective elimination of sperm mtDNA in imraspecies crosses, even though the sperm mitochondria contribute to the zygote at fertilisation. Therefore all children of a female are clonal for mtDNA. The clonal maternal transmission and an increased mutation rate contribute to accumulation of new mtDNA mutations within the population.

Homoplasy refers to the presence of identical mtDNA found normally in an individual cell. By comparison, heteroplasmy is the coexistence of mitochondria containing differing genomic sequences (wild-type and mutated mtDNA) in the same cell. Heteroplasmy may imply the presence of a harmful mutation in mtDNA although benign heteroplasmic polymorphisms also exist. During cell division, mtDNA replicates itself, but unlike nuclear genes, the new mitochondrial molecules segregate passively to daughter cells. A mitochondrial mutation is a random change in a single molecule. Over time, chance segregation leads to proliferation of mutants in a cell. Differing levels of heteroplasmy for individual mitochondrial mutations are found in various tissues due to passive segregation of mtDNA at cell division. In humans mitochondrial heteroplasmy is associated with disease. The severity of some pathogenic conditions such as neuromuscular disorders correlates with a high number of mutant mtDNA in skeletal muscle (post mitotic tissue). Therefore a range of clinical phenotypes from asymptomatic to severely affected is associated with varying levels of heteroplasmy within a pedigree.

In some tissues and organs selection might operate and lead to a reduced or increased level of mutant DNA. In this model, negative selection will decrease the level of a mutant over time whereas levels of a mutant mtDNA can increase during life in particular tissues. In some tissues high levels of mutation load have been associated with disease progression. Defective mitochondria with an increased mutation load may selectively proliferate (perhaps as part of a compensatory process) in response to a respiratory chain defect. This positive selection may lead to increased levels of mutant mtDNA within a post-mitotic tissue such as muscle.

A special feature of mitochondrial disease is the heterogeneity found in the clinical condition from single organs to multisystem disease. Over 50 pathogenic mtDNA base substitution mutations are known. The base substitutions are divided into both missense mutations affecting the 13 protein encoding genes and those affecting the rRNA and tRNA with global effects on mitochondrial protein synthesis. Many more mtDNA rearrangements (deletions and insertions) have been found in a variety of degenerative disorders.

Most frequently reported mitochondrial mutations are A3243G present in MELAS (myopathy, enchephalopathy, lactic acidosis and stroke-like episodes), AS344G in MERFF (myoclonus epileplsy, ragged red fibres), T8993G/C in NARP (neuropathy, ataxia, neuritis pigmentosa), Leber's inherited optic neuropathy (LHON) and Leigh syndrome. Mutations in the nuclear genome could also affect mitochondrial function by inactivating the OXPHOS system or destabilising the mtDNA. For example in Friedreich's ataxia.

There is accumulating evidence indicating that the involvement of mitochondrial mutations leading to inherited disorders may underlie a large percentage of the common, late-onset, heterogeneous disorders such as sensorineural impairment and diabetes and other age-related disorders. Table 1 lists a range of well characterised clinical disorders which may be linked to a variety of mtDNA mutations.

**Table 1.**

| **MtDNA Point mutations** | **Single deletion or duplication** | **Multiple deletions** | **Depletion of mtDNA** | **Several types of mtDNA defect** |
|---|---|---|---|---|
| Cardiomyopathy | Ataxia | Aging | Infantile | Deafness |
| LHON | Leukodystrophy | Myositis | Myopathy, | DM |
| MELAS | DM | MNGIE | AZT treatment | PEO |
| MERRF | Kearns-Sayre | | | Leigh's syndrome |
| Leigh's syndrome | syndrome | | | Myopathy |
| NARP | Pearson's | | | Rhabdomyolysis |
| | PEO | | | Sensory neuropathy |

The inventors formed a hypothesis that the development of conditions at least partially characterised by inappropriate fibrosis or scarring (e.g. DD) may be linked to mutations or polymorphisms in the mitochondrial genome. The hypothesis was based upon the realisation that some DD patients demonstrate maternally inherited disease. As mitochondrial disorders are maternally inherited, they decided to test their hypothesis by comparing mtDNA from subjects with DD and control subjects.

The inventors performed experiments to screen the mitochondrial genome for polymorphisms or mutations that may be associated with a condition at least partially characterised by inappropriate fibrosis or scarring. Having established such an association the inventors have established that DNA taken from a subject may be analysed to help establish a diagnosis of the condition or to establish whether or not a subject is predisposed to develop such a condition.

The condition may be a form of pathological scarring of the skin (e.g. hypertrophic scarring or keloids) or an internal scar or fibrosis as mentioned above. Alternatively the condition may be a fibrotic disease or disorder also as mentioned above.

The method of the invention may be used to help diagnose or detect fibrotic disorders of the skin such as:-
Scleroderma
Systemic sclerosis
Crest Syndrome
Tuberous sclerosis with skin patches
Familial cutaneous collagenoma
Metabolic and immunologic disorders of the skin (porphyria cutanea tarda, chronic graft versus host disease)
Eosinophilic facsitis
Discoid lupus erythematosus, Dermatomyositis
Mixed connective tissue disease
Drug-induced skin fibrosis - bleomysin, PVC, silicates
Peyronies
Oral submucous fibrosis
Fibrosis induced following dietary and environmental exposures.

The method is also useful in the diagnosis or detection of fibrotic disorders of other organs. These include:-
Pulmonary/cardiac fibrosis
Liver fibrosis/cirrhosis
Renal fibrosis
GI tract fibrosis
Drug induced fibrosis (e.g. post organ transplantation)
Central and peripheral nervous system fibrosis
Vascular system (veins and arteries) fibrosis
Male & female genitourinary tract fibrosis
Gynaecological (fallopian tube fibrosis, uterine fibromas)

The method of the first aspect of the invention is particularly suited for diagnosing or detecting a predisposition to Dupuytren's Disease (DD).

Studies were performed (as outlined in more detail in the Examples) on mtDNA isolated from myofibroblasts. The myofibroblast has been demonstrated in many studies to be a key cell responsible for tissue contraction in DD and has also been reported to contain a large number of mitochondria.

The inventors used Denaturing High performance Liquid Chromatography (DHPLC) in conjunction with the Transgenomic Wave Nucleic Acid Fragment Analysis System (WAVE System) to investigate polymorphisms or mutations in the mitochondrial genome. These apparatus may be used in a highly efficient, cost effective and automated technique for detection of unknown mutations (Jones et al., Human Mutation 2001; 17(3): 233-234.). The technique utilises a commercially available divynyl benzene bead matrix (DNASep, Transgenomic Inc, Ne, USA) and ion paired reverse phase HPLC to detect heteroduplex DNA species present in samples containing a mutation, which form subsequent to PCR amplification. Differential elution of hetero and homoduplexes can occur by the application of partially denaturing conditions (e.g. by temperature elevation). At a point where the DNA starts to melt the difference in helical content of the homo and heteroduplex species may be maximised thus changing the degree to which each species binds to the matrix. This differential elution of homo and heteroduplex species leads to a change in chromatogram pattern and hence the identification of the presence of a mutation. Importantly, unlike other techniques, DHPLC is capable of detecting extremely low levels of mutant within a wild type population. This capability of DHPLC makes it suitable for scanning the mitochondrial genome.

As illustrated in Example 1, the inventors found that certain polymorphisms or mutations in mtDNA significantly correlated with the development of conditions at least partially characterised by inappropriate fibrosis or scarring. Examples of preferred polymorphisms or mutations that may be detected according to the invention are found at the following positions in the mitochondrial genome:

### (a) Mutations in the 16srRNA region of the mitochondrial genome

The inventors found that mutations in the 16rRNA region of the mitochondrial genome correlated with conditions according to the method of the invention.

The mutation may be contained within a 342 bp fragment (i.e. positions 3192-3533 of the genome) of the 16srRNA gene with the following nucleotide sequence:

TTAGTATTATACCCACACCCACCCAAGAACAGGGTTTGTTAAGATGGCAG
AGCCCGGTAATCGCATAAAACTTAAAACTTTACAGTCAGAGGTTCAATTC
CTCTTCTTAACAACATACCCATGGCCAACCTCCTACTCCTCATTGTACCCA
TTCTAATCGCAATGGCATTCCTAATGCTTACCGAACGAAAAATTCTAGGCT
ATATACAACTACGCAAAGGCCCCAACGTTGTAGGCCCCTACGGGCTACTA
CAACCCTTCGCTGACGCCATAAAACTCTTCACCAAAGAGCCCCTAAAACC
CGCCACATCTACCATCACCCTCTACATCACCGCCCCGACC

(SEQ ID No. 1)

Alternatively the mutation may be contained within a 442 bp fragment (i.e. positions 2415-2856 of the genome) of the 16srRNA gene with the following nucleotide sequence:

ctcactgtcaacccaacacaggCATGCTCATAAGGAAAGGTTAAAAAAAGTAAAAGGA
ACTCGGCAAATCTTACCCCGCCTGTTTACCAAAAACATCACCTCTAGCATC
ACCAGTATTAGAGGCACCGCCTGCCCAGTGACACATGTTTAACGGCCGCG
GTACCCTAACCGTGCAAAGGTAGCATAATCACTTGTTCCTTAAATAGGGA
CCTGTATGAATGGCTCCACGAGGGTTCAGCTGTCTCTTACTTTTAACCAGT
GAAATTGACCTGCCCGTGAAGAGGCGGGCATAACACAGCAAGACGAGAA
GACCCTATGGAGCTTTAATTTATTAATGCAAACAGTACCTAACAAACCCA
CAGGTCCTAAACTACCAAACCTGCATTAAAAATTTCGGTTGGGGCGACCT
CGGAGCAGAACCCAACCTCCGAGCAGTACATGC

(SEQ ID No. 2)

A most preferred mutation is found at position 2839 of the mitochondrial genome. This mutation represents an insertion of a thymidine nucleotide (T) after the Guanosine nucleotide (G) (G2839G/T). Example 1 illustrates that 16/18 samples from DD sufferers had the GT genotype whereas all control (CW) had the wildtype (G) genotype.

### (b) Mutations in polypeptides of Complex I of the Respiratory Chain

The NADH-quinone oxidoreductase (complex I) is one of three energy-transducing enzyme complexes of the respiratory chain in mitochondria. It is the point of entry for the major fraction of electrons that traverse the respiratory chain eventually resulting in the reduction of oxygen. Complex I is presumed to be one of the most intricate membrane-bound enzymes known to date, being composed of at least 43 unlike polypeptides. Of these, 7 are encoded by mtDNA (ND 1, 2, 3, 4, 4L, 5 and 6).

The mutation may be contained within a 179 bp fragment (i.e. positions 3429-3607 of the genome) of the ND1 gene with the following nucleotide sequence:

ccctacgggctactacaacccTTCGCTGACGCCATAAAACTCTTCACCAAAGAGCCCC
TAAAACCCGCCACATCTACCATCACCCTCTACATCACCGCCCCGACCTTAG
CTCTCACCATCGCTCTTCTACTATGAACCCCCCTCCCCATACCCAACCCCC
TGGTCAACCTCAACCTAGG

(SEQ ID No. 3)

Alternatively the mutation may be contained within a 242 bp fragment (i.e. positions 3608-3849 of the genome) of the ND1 gene with the following nucleotide sequence:

CCTCCTATTTATTCTAGCCACCTCTAGCCTAGCCGTTTACTCAATCCTCTGA
TCAGGGTGAGCATCAAACTCAAACTACGCCCTGATCGGCGCACTGCGAGC
AGTAGCCCAAACAATCTCATATGAAGTCACCCTAGCCATCATTCTACTATC
AACATTACTAATAAGTGGCTCCTTTAACCTCTCCACCCTTATCACAACACA
AGAACACCTCTGATTACTCCTGCCATCATGACCCTTGG

(SEQ ID No. 4)

Alternatively the mutation may be contained within a 470 bp fragment (i.e. positions 3959-4428 of the genome) of the ND1 gene with the following nucleotide sequence:

CCCCTTCGCCCTATTCTTCATAGCCGAATACACAAACATTATTATAATAAA
CACCCTCACCACTACAATCTTCCTAGGAACAACATATGACGCACTCTCCCC
TGAACTCTACACAACATATTTTGTCACCAAGACCCTACTTCTAACCTCCCT
GTTCTTATGAATTCGAACAGCATACCCCCGATTCCGCTACGACCAACTCAT
ACACCTCCTATGAAAAAACTTCCTACCACTCACCCTAGCATTACTTATATG
ATATGTCTCCATACCCATTACAATCTCCAGCATTCCCCCTCAAACCTAAGA
AATATGTCTGATAAAAGAGTTACTTTGATAGAGTAAATAATAGGAGCTTA
AACCCCCTTATTTCTAGGACTATGAGAATCGAACCCATCCCTGAGAATCCA
AAATTCTCCGTGCCACCTATCACACCCCATCCTAAAGTAAggtcagctaaataagcta
tcggg

(SEQ ID No. 5)

A striking DHPLC pattern was found in the ND2 region in 94 % of cases that was only present in 45 % of controls. This indicated a mutation was present with linkage to conditions according to the invention.

Two further novel mutations in the ND2 region (at positions A4916G and G5045A) were found in DD subjects and may be detected in a test according to the present invention.

The mutation may be contained within a 396 bp fragment (i.e. positions 4846-5241 of the genome) of the ND2 gene with the following nucleotide sequence:

CGGCCTGCTTCTTCTCACATGACAAAAACTAGCCCCCATCTCAATCATATA
CCAAATCTCTCCCTCACTAAACGTAAGCCTTCTCCTCACTCTCTCAATCTT
ATCCATCATAGCAGGCAGTTGAGGTGGATTAAACCAAACCCAGCTACGCA
AAATCTTAGCATACTCCTCAATTACCCACATAGGATGAATAATAGCAGTTC
TACCGTACAACCCTAACATAACCATTCTTAATTTAACTATTTATATTATCCT
AACTACTACCGCATTCCTACTACTCAACTTAAACTCCAGCACCACGACCCT
ACTACTATCTCGCACCTGAAACAAGCTAACATGACTAACACCCTTAATTCC
ATCCACCCTCCTCTCCCTAGGAGGCCTGCCCCCGCTAAC

(SEQ ID No. 6)

Alternatively the mutation may be contained within a 531 bp fragment (i.e. positions 4180 - 4710 of the genome) of the ND2 gene with the following nucleotide sequence:

acttcctaccactcaccctagcATTACTTATATGATATGTCTCCATACCCATTACAATCT
CCAGCATTCCCCCTCAAACCTAAGAAATATGTCTGATAAAAGAGTTACTTT
GATAGAGTAAATAATAGGAGCTTAAACCCCCTTATTTCTAGGACTATGAG
AATCGAACCCATCCCTGAGAATCCAAAATTCTCCGTGCCACCTATCACACC
CCATCCTAAAGTAAGGTCAGCTAAATAAGCTATCGGGCCCATACCCCGAA
AATGTTGGTTATACCCTTCCCGTACTAATTAATCCCCTGGCCCAACCCGTC
ATCTACTCTACCATCTTTGCAGGCACACTCATCACAGCGCTAAGCTCGCAC
TGATTTTTTACCTGAGTAGGCCTAGAAATAAACATGCTAGCTTTTATTCCA
GTTCTAACCAAAAAAATAAACCCTCGTTCCACAGAAGCTGCCATCAAGTA
TTTCCTCACGCAAGCAACCGCATCCATAATCCTTCTAATAGCTATCCTCTT
CAACAATATACTCTC

(SEQ ID No. 7)

### (c) Mutations in polypeptides of Complex III (Cytochrome b) of the Respiratory Chain

Multiple novel mutations were found in DD sufferers that showed a very similar DHPLC pattern between at least two subjects. These sequence changes were not present in any controls. The majority of changes found by sequencing revealed the novel presence of heteroplasmy which included G15274C/G; A15282C/A; G15319C/G; T15332C/T; G15336G/C; T15339T/C; T15362C/T and 15434insC. All these changes were in the Cytochrome b region.

### (d) Mutations in polypeptides of Complex IV (Cytochrome c oxidase) of the Respiratory Chain

A prominent DHPLC pattern change was found in samples from DD suffers within 6688 and 6849 of the genome (the CO I region). All DD subjects had a single peak DHPLC pattern whereas only 27 % of the control samples had a similar pattern.

The mutation may be contained within a 162 bp fragment (i.e. positions 6688-6849of the genome) of the CO I gene with the following nucleotide sequence:

CGGAAAAAAAGAACCATTTGGATACATAGGTATGGTCTGAGCTATGATAT
CAATTGGCTTCCTAGGGTTTATCGTGTGAGCACACCATATATTTACAGTAG
GAATAGACGTAGACACACGAGCATATTTCACCTCCGCTACCATAATCATC
GCTATCCCCA
(SEQ ID No. 8)

Further work has established that subjects with other fibrotic disorders (e.g.scleroderma or renal fibrosis) and subjects liable to develop severe/pathological scarring (e.g. keloids) also have a high frequency of these mutations. Therefore the polymorphism or mutation may be examined according to the method of the invention for diagnosing or detecting a predisposition to a variety of conditions at least partially characterised by inappropriate fibrosis or scarring.

Various techniques may be used to detect polymorphisms or mutations according to the method of the invention.

One preferred technique is DHPLC. The technique is described in more detail in the Example. The DHPLC technique of Van Den Bosch et al. (Nucleic Acids Res 2000 Oct 15; 28(20): E89) may be used, or adapted, for use according to the method of the invention.

Another preferred technique involves Restriction Enzyme Digestion (RED) and is based upon the fact that polymorphisms or mutations can lead to the production of different sized DNA fragments following treatment with a restriction enzyme (because of the introduction or deletion of a restriction site). These fragments may be visualised on gels and the polymorphism identified based upon the number and size (i.e. distance moved on the gel) of the fragments from a DNA sample derived from a subject.

It is preferred that the mtDNA is isolated and then amplified prior to detection of the polymorphism or mutation. This amplification is preferably by means of the polymerase chain reaction (PCR). For instance a preferred method according to the invention known as the PCR-restriction fragment length polymorphism method (PCR-RFLP) and involves PCR amplification of DNA containing the polymorphism or mutation prior to RED and subsequent analysis. For some polymorphisms neither the wild type nor the mutant allele abolishes or introduces a restriction enzyme site. When this is the case, a restriction enzyme site may be introduced by specifically designing PCR primers that introduce restriction sites into the amplified product. The introduced enzyme site allows differentiation between polymorphic alleles and wild type by size analysis. For example if the restriction products of the amplified product are analysed by gel electrophoresis (agarose or polyacrylamide gel, for example) the alleles with the introduced restriction enzyme site will produce an extra band on the gel.

Other techniques that may be used to detect polymorphisms according to the present invention include :-
(1) Direct sequencing of the polymorphic region of interest (e.g. using commercially available kits such as the Cy5™ Thermo Sequenase^{™} dye terminator kit - Amersham Pharmacia Biotech);
(2) Sequence Specific Oligonucleotide Hybridization (SSO) (involving dot or slot blotting of amplified DNA molecules comprising the polymorphic region; hybridisation with labelled probes which are designed to be specific for each polymorphic variant; and detection of said labels);
(3) Heteroduplex and single-stranded conformation polymorphism (SSCP) Analysis (involving analysis of electrophoresis band patterns of denatured amplified DNA molecules comprising the polymorphic region);
(4) Sequence Specific Priming (SSP) [also described as Amplification Refractory Mutation System (ARMS)];
(5) Mutation Scanning [e.g. using the PASSPORT^{™} Mutation Scanning Kit (Amersham Pharmacia Biotech)];
(6) Chemical Cleavage of Mismatch Analysis;
(7) Non-isotopic RNase Cleavage Assay (Ambion Ltd.);
(8) Enzyme Mismatch Cleavage Assay; and
(9) Single Nucleotide Extension Assay.

When PCR amplification is required, PCR primers need to be designed such that they are suitable for amplifying a region around the relevant polymorphism or mutation.

PCR Primer set 6 (see Example 1 and SEQ ID No. 9 and SEQ ID No. 10 below) are preferred primers for detecting mutations in the 16srRNA gene. Amplification with these primers may be followed by Ddel restriction enzyme digestion (to generate smaller DNA fragments) as described in Example 1.
*Primer set 6 forward primer: 5'* ctc act gtc aac cca aca cag g 3' (SEQ ID No. 9)
*Primer set 6 reverse primer: 5'* tgt gtt gtg ata agg gtg gag ag 3' (SEQ ID No. 10)

Suitable primers for specifically amplifying the mutation at 2839 are listed below as SEQ ID No. 11 and SEQ ID No. 12.
*Forward primer: 5'* tgc att aaa aat ttc ggt tgg *3'* (SEQ ID No. 11) (Length 21bp; No GC 7; No AT 14; %GC 33%; Tm = 48.81)
*Reverse primer: 5'* tgt cct gat cca aca tcg ag *3'* (SEQ ID No. 12) (Length 20bp; No GC 10; No AT 10; %GC 50%; Tm 54.22)

Primers of SEQ ID No. 11 and SEQ ID No. 12 may be used to amplify the region of the mitochondrial genome containing the 2839 mutation. The amplified mtDNA may then be sequenced to identify the genotype (i.e. a direct sequencing technique).

mtDNA may be isolated from blood or tissue samples (e.g. hair, oral buccal swabs, nail or skin) or from other suitable sources using conventional methods. Preferably the DNA is isolated from whole blood or granulocytes, palmar fascia, plantar fascia or penile fascia. Myofibroblasts also represent a preferred source of mtDNA for testing according to the method of the invention.

The method is preferably used to examine mtDNA from a human subject. However it will be appreciated that DNA derived from animal subjects of vetinary interest may also be tested according to the method of the invention.

A prediction or diagnosis based upon the method according to the present invention depends upon an association being made between a particular condition and the specific polymorphism or mutation in question. Such associations were established by the inventors by performing further experiments and making statistical analyses. Provision of data based upon association analyis enables a clinician to interpret the significance of genotypes identified by sequencing DNA according to the method of the invention. The clinician may then make a judgement regarding the likelihood of a patient developing, or having, a particular disease or disorder. Such knowledge is important in the clinical management of specific conditions associated with inappropriate scarring or fibrosis. It will be appreciated that data relating to the association of a particular genotype with a condition may be provided to a user of the method according to the invention (e.g. a technician or clinician) by incorporating a data sheet as part of a kit (see below).

Genetic testing may be carried out either pre-natally, peri-natally or post-natally when it is desired to test whether or not a neonate or child is likely to have inherited a predisposition to develop a condition at least partially characterised by inappropriate fibrosis or scarring. This is particularly useful when there is believed to be a family history of developing the condition.

The test is particularly useful for testing subjects pre-operatively. The results of such a test are useful for establishing whether or not there could be healing complications for the subject undergoing surgery (e.g. hypertrophic scarring, keloids or internal fibrosis/scarring).

The test is also useful before a therapeutic regimen is established for treating a condition characterised by inappropriate scarring or fibrosis. The results of the test according to the invention may be used by a clinician to help in the selection of medicaments used and the dosage thereof.

It is most preferred that the method of the invention is used to test subjects with a family history of developing a condition at least partially characterised by inappropriate scarring or fibrosis.

The various elements required for a technician to perform the method of the first aspect of the invention may be incorporated in to a kit. Thus according to a second aspect of the present invention there is provided a kit comprising:
A) PCR primers for amplifying genetic polymorphisms in the 16srRNA of the mitochondrial genome that are linked to a condition characterised by inappropriate scarring or fibrosis;
B) Control DNA samples of known genotype for each polymorphism, and
C) A data card outlining linkage between a particular polymorphism and a condition at least partially characterised by inappropriate fibrosis or scarring.

It is preferred that the kit comprises primers specific for a target sequence of sample DNA known to contain a polymorphism or mutation of interest. For instance, suitable PCR primers for a kit for genotyping the G2838G/T mutation are the primers of SEQ ID No. 9 and SEQ ID No. 10 or the primers of SEQ ID No. 11 and SEQ ID No. 12.

The kit may further comprise:
D) A suitable restriction enzyme for generating fragments of the mtDNA sample;
E) Protocols for PCR amplification, restriction enzyme digestion of PCR products and agarose gel electrophoresis of DNA fragments; and
F) Relevant buffers.

Buffers provided with the Kit may be in liquid form and preferably provided as pre-measured aliquots. Alternatively the buffers may be in concentrated (or even powder form) for diluting.

The Kit may further comprise suitable reaction vessels, centrifuge tubes etc.

The inventors have found that modulators of mitochondrial genome gene products may be used to treat each of the conditions referred to in relation to the first aspect of the invention. It is preferred that the modulators are used to treat Dupuytren's Disease (DD).

Several classes of compounds may be used as such modulators. These compounds include:
(i) inhibitors or activators of the mitochondrial gene products (e.g. allosteric or competitive inhibitors);
(ii) compounds which regulate synthesis of mitochondrial gene products;
(iii) compounds which regulate release of mitochondrial gene products;
(iv) compounds which regulate the rate of inactivation or metabolism of mitochondrial gene products;
(v) compounds which regulate mitochondrial gene product expression and/or transcription (e.g. ribozymes or antisense DNA molecules); and
(vi) antibodies or intrabodies raised against the mitochondrial gene products.

It is preferred that the gene product modulated is Complex I of the respiratory chain; Complex III (Cytochrome b) of the respiratory chain; or Complex IV (Cytochrome C Oxidase) of the respiratory chain. It is most preferred that the gene product modulated is 16srRNA.

Preferred antisense molecules, antibodies and intrabodies may be raised against any one of SEQ ID NOs. 1 - 12.

The modulators may be used to treat existing conditions but may also be used when prophylactic treatment is considered medically necessary. For instance, when it is considered necessary to initiate therapy before elective surgery to prevent development of hypertrophic scarring or keloids.

The modulators may take a number of different forms depending, in particular on the manner in which they are to be used. Thus, for example, composition may be in the form of a powder, tablet, capsule, liquid, ointment, cream, gel, hydrogel, aerosol, spray, micelle, transdermal patch, liposome or any other suitable form that may be administered to a person or animal. It will be appreciated that the vehicle of the composition of the invention should be one which is well tolerated by the subject to whom it is given and enables delivery of the compounds to the subject.

The modulators may be used in a number of ways. For instance, systemic administration may be required in which case the modulator may be contained within a composition which may, for example, be ingested orally in the form of a tablet, capsule or liquid. Alternatively the modulation may be administered by injection into the blood stream. Injections may be intravenous (bolus or infusion) or subcutaneous (bolus or infusion). The modulators may also be administered by inhalation (e.g. intranasally).

It is preferred that the modulators are for topical administration to the tissue affected by, or expected to be affected by, the condition.

The modulator may also be incorporated within a slow or delayed release device. Such devices may, for example, be inserted on or under the skin and the modulator may be released over weeks or even months. Such a device may be particularly useful for patients with chronic conditions The devices may be particularly advantageous when a modulator is used which would normally require frequent administration.

It will be appreciated that the amount of a modulator required is determined by biological activity and bioavailability which in turn depends on the mode of administration, the physicochemical properties of the compound employed and whether the modulator is being used as a monotherapy or in a combined therapy. The frequency of administration will also be influenced by the abovementioned factors and particularly the half-life of the modulator within the subject being treated.

Known procedures, such as those conventionally employed by the pharmaceutical industry (e.g, *in vivo* experimentation, clinical trials etc), may be used to establish specific formulations of compositions and precise therapeutic regimes (such as daily doses of the modulator and the frequency of administration).

A preferred means of using protein or peptide modulators is to deliver the modulator to the affected tissue by means of gene therapy. Such means may be a delivery system for use in a gene therapy technique, said delivery system comprising a DNA molecule encoding for a protein which directly or indirectly modulates mitochondrial genome gene products, said DNA molecule being capable of being transcribed to allow the expression of said protein and thereby treating a condition at least partially characterised by inappropriate fibrosis or scarring.

Such delivery systems are highly suitable for achieving sustained levels of a protein which directly or indirectly modulate mitochondrial genome gene products over a longer period of time than is possible for most conventional therapeutic regimes. The delivery system may be used to induce continuous protein expression from cells that have been transformed with the DNA molecule. Therefore, even if the protein has a very short half-life as an agent *in vivo*, therapeutically effective amounts of the protein may be continuously expressed from the treated tissue.

Furthermore, the delivery system may be used to provide the DNA molecule (and thereby the protein which is an active therapeutic agent) without the need to use conventional pharmaceutical vehicles such as those required in tablets, capsules or liquids.

The delivery system is such that the DNA molecule is capable of being expressed (when the delivery system is administered to a patient) to produce a protein which directly or indirectly has activity for modulating mitochondrial gene product activity. By "directly" we mean that the product of gene expression *per se* has the required activity. By "indirectly" we mean that the product of gene expression undergoes or mediates (e.g. as an enzyme) at least one further reaction to provide a modulator effective treating the condition.

The DNA molecule may be contained within a suitable vector to form a recombinant vector. The vector may for example be a plasmid, cosmid or phage. Such recombinant vectors are highly useful in the delivery systems of the invention for transforming cells with the DNA molecule.

Recombinant vectors may also include other functional elements. For instance, recombinant vectors can be designed such that the vector will autonomously replicate in the cell. In this case, elements which induce DNA replication may be required in the recombinant vector. Alternatively the recombinant vector may be designed such that the vector and recombinant DNA molecule integrates into the genome of a cell. In this case DNA sequences which favour targeted integration (e.g. by homologous recombination) are desirable. Recombinant vectors may also have DNA coding for genes that may be used as selectable markers in the cloning process.

The recombinant vector may also further comprise a promoter or regulator to control expression of the gene as required.

The DNA molecule may (but not necessarily) be one which becomes incorporated in the DNA of cells of the subject being treated. Undifferentiated cells may be stably transformed leading to the production of genetically modified daughter cells (in which case regulation of expression in the subject may be required e.g. with specific transcription factors or gene activators). Alternatively, the delivery system may be designed to favour unstable or transient transformation of differentiated cells in the subject being treated. When this is the case, regulation of expression may be less important because expression of the DNA molecule will stop when the transformed cells die or stop expressing the protein (ideally when the condition has been treated or prevented).

The delivery system may provide the DNA molecule to the subject without it being incorporated in a vector. For instance, the DNA molecule may be incorporated within a liposome or virus particle. Alternatively the "naked" DNA molecule may be inserted into a subject's cells by a suitable means e.g. direct endocytotic uptake.

The DNA molecule may be transferred to the cells of a subject to be treated by transfection, infection, microinjection, cell fusion, protoplast fusion or ballistic bombardment. For example, transfer may be by ballistic transfection with coated gold particles, liposomes containing the DNA molecule, viral vectors (e.g. adenovirus) and means of providing direct DNA uptake (e.g. endocytosis) by application of the DNA molecule directly to the brain topically or by injection.

It is preferred that methods designed for targeting mitochondrial originating conditions are used to implement the treatments described herein. For instance, an antigenomic approach may be adopted using peptide nucleic acids (PNA). Endogenous cell replacement therapy and allotropic gene therapy may also be employed to treat mtDNA diseases. Such therapy has recently been reviewed by Turnbull & Lightowlers (Nat Genet 2002 April 30(4): 345-346) and it will be appreciated that the therapeutic techniques disclosed therein (and incorporated herein by reference) may be used in preferred methods of treatment according to the present invention.

The invention will be further described, by way of example only, with reference to the accompanying drawings, in which:
Figure 1 is a schematic representation of the modified DHPLC technique of Van Den Bosch et al. (Nucleic Acids Res 2000 Oct 15; 28(20): E89) *supra*) as used in Example 1;
Figure 2 is a schematic representation of the mitochondrial genome showing the position of primer sets used for amplification in Example 1;
Figure 3 illustrates primer set 2 amplification of a 331bp product from the mitochondrial genome showing the increased fidelity of the Optimase polymerase used in Example 1 in comparison to Expand DNA polymerase (Roche) wherein the traces within the graph from top to bottom represent Expand 2, Expand 2, Pho 12 and Pho 12;
Figure 4 is a graph illustrating DHPLC mutants identified using Primer set 4 in fragment 3; in DD7 and DD12 compared to WT pattern in DD1 wherein the traces within the graph from top to bottom represent mutants in DD7, DD12 and DD1, respectively;
Figure 5 is a graph illustrating DHPLC mutants identified using Primer set 4 in fragment 3 in Example 1 wherein the traces within the graph from top to bottom represent CW 19 compared to CW 13;
Figure 6 is a graph illustrating DHPLC mutants identified using Primer set 5 in Example 1 wherein the traces within the graph from top to bottom represent DD7, DD1 and CW3;
Figure 7 is a graph illustrating DHPLC for mutation at 1690bp identified using Primer set 5 in Example 1 wherein the traces within the graph from top to bottom represent DD1, CW19 and DD7;

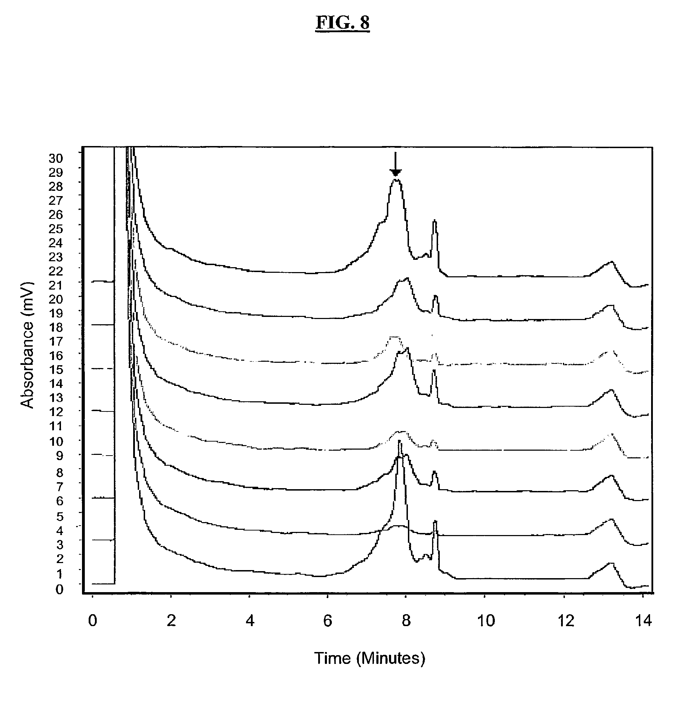

Figure 8 is a graph illustrating DHPLC for mutation at 1811bp identified using Primer set 5 in Example 1 wherein the traces within the graph from top to bottom represent DD6, DD12, DD20, CW3, CW4, CW10, CW12 and DD7;
Figure 9 is a graph illustrating an affected versus a control sample with the mutation being evident as an enhanced double peak pattern in fragment 5 identified using Primer set 6 of Example 1 wherein the traces within the graph from top to bottom represent DD5 and CW3;
Figure 10 is a graph illustrating DHPLC mutants identified using primer set 7 in Example 1 wherein the traces within the graph from top to bottom represent DD1, DD2, DD 11, DD14, DD 17, DD18 and DD5;
Figure 11 is a graph illustrating DHPLC mutants identified using primer set 8 in Example 1;
Figure 12 is a graph illustrating DHPLC mutants identified using primer set 9 in fragment 4; in DD12, CW3 and CW10 in Example 1;
Figure 13 is a graph illustrating a one-peak pattern found in all DD samples whereas only 83% of controls (CW) have the one peak pattern identified using primer set 10 in Example 1 wherein the traces within the graph from top to bottom represent CW5 and DD1; and
Figure 14 is a graph illustrating DHPLC restriction site mutants identified using Primer set 16 in CW4 and CW6 in Example 1.

### EXAMPLE 1: IDENTIFICATION OF MUTATIONS IN mtDNA LINKED WITH DUPUYTREN'S DISEASE

Samples were taken from a group of patients with Dupuytren's Disease (DD) and a control group to investigate the correlation between polymorphism and the condition.

### 1.1 METHODS

The inventors modified the DHPLC technique of Van Den Bosch et al. (Nucleic Acids Res 2000 Oct 15; 28(20): E89) to scan the mitochondrial genome for mutations that may be linked to DD.

### 1.1.1 Modifications to the DHPLC technique of Van Den Bosch et al.

The inventors modified the DHPLC technique of Van Den Bosch *et al. supra*) to enable mitochondrial genome mutation scanning of large sample populations to be accomplished more quickly at reasonable cost. The changes made are outlined in Table 2.

**Table 2**

| | **Protocol detail** | **Transgenomic** | **Smeets** |
|---|---|---|---|
| 1 | PCR primer sets required | 18 | 13 |
| 2 | Restriction enzymes required | 5 | 10 |
| 3 | Restriction digests required | 15 | 13 |
| 4 | Number of DHPLC analysis | 29 | 65 |
| 5 | Enzyme recommended | Optimase (Transgenomic) | Taq (ABI) |

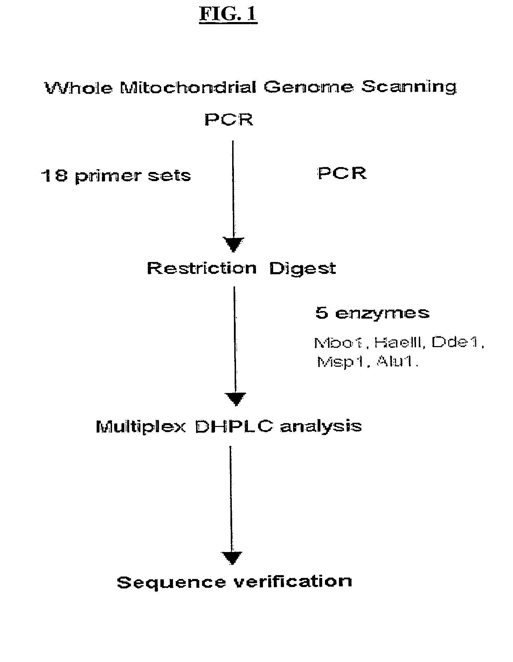

A schematic representation of the process, as modified over Van Den Bosch *et al*. is illustrated in Figure 1. Figure 1 shows a PCR amplification using Optimase DNA polymerase of the entire mitochondrial genome using a set of 18 specifically designed primers. Four fragments from the amplification set are used in routine DHPLC but the remaining fourteen undergo restriction digestion to produce a range of fragments, which can then be subjected to multiplex DHPLC using a total of 29 different parameters (gradient and temperature). Positive samples are indicated by a change in chromatogram pattern and the mutations present verified by sequencing.

### 1.1.1(a) The importance of primer design

The improvements made to the DHPLC technique of Van Den Bosch *et al.* all originate from the primer design which were designed with the following criteria in mind:
1. Individual fragments for DHPLC (non multiplex) of the hypervariable region of the mitochondrial genome.
2. Consideration of the individual major coding genes.
3. Consideration of known disease causing mutations.

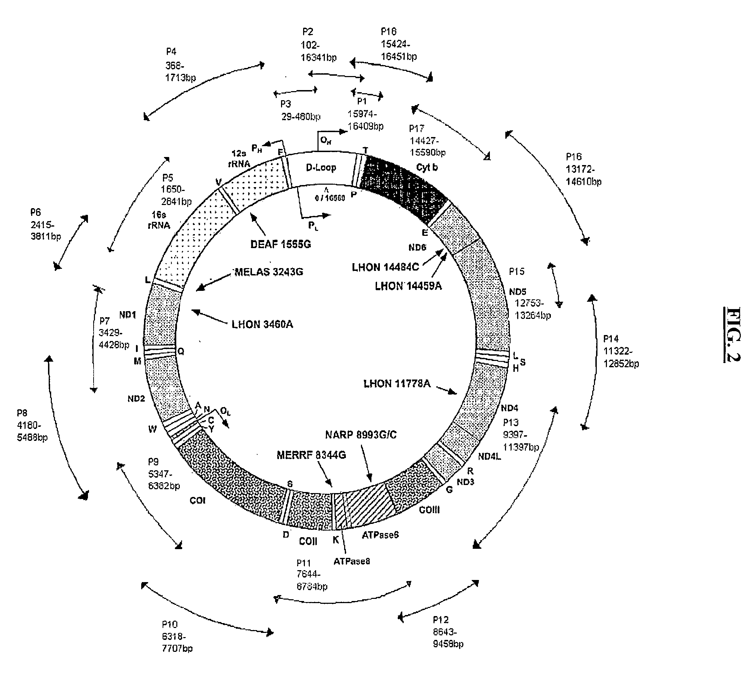

The primer positions and these criteria are illustrated in Figure 2. As regards to scanning the hypervariable region, 3 overlapping PCR amplifications were used instead of one large fragment with subsequent restriction enzyme digestion as in the original technique. The hypervariable, although non coding, nature of this region lead to high numbers of positive DHPLC patterns which were verified by sequencing using this strategy.

Although a larger number of PCRs were required for whole genome amplification than used in the original technique, we believe this was worthwhile because fewer restriction enzymes (5 instead of 10) needed to be purchased and more importantly less DHPLC scanning conditions needed to be employed. The decrease in scanning conditions from 65 to 29 was a real time and cost saving element, which is accentuated with the need to scan large numbers of samples in a research setting as undertaken with this project.

### 1.1.1(b) The importance of PCR enzyme choice

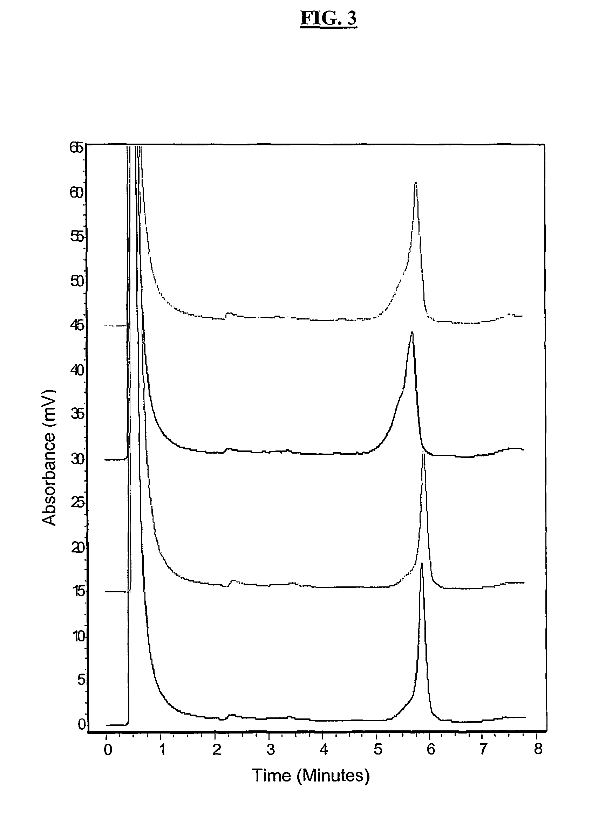

Due to the heteroplasmic nature of mitochondrial populations, a mutation detection technique must have sufficient sensitivity to find small proportions of a mutation in a predominantly wild type population. With this in mind, a key criterion for this application is high fidelity in sample preparation by PCR amplification. All Taq DNA polymerase enzymes have an inherent level of misincorporation dependent on their origin and method of use. Each misincorporation during PCR amplification is the equivalent of a random low-level heteroduplex, which may be visualised with the DHPLC technique. It is therefore advisable to maintain these misincorporations at the lowest level possible and hence obtain the greatest chance of success. We have substituted a novel proof reading polymerase (Optimase DNA polymerase, Transgenomic Inc), which has been shown to have extremely high levels of fidelity. Figure 3 shows the WT pattern obtained for primer set 2 using Optimase polymerase in comparison to Expand DNA polymerase (Roche). The sharper peak pattern obtained under partially denaturing conditions using the Optimase DNA polymerase is indicative of the increased fidelity of the enzyme.

### 1.1.2 Experimental Procedure

### 1.1.2(a) Sample collection

**Cases:** Dupuytren's disease (DD) patients with maternal inheritance (n =20) were enrolled in the study. The age range was between 37 to 90 years with a mean age of 56.9 years. Cases were all unrelated Caucasians from the Northwest region of England; U.K. Dupuytren's cases were mostly identified through operative record clinical codes from the South Manchester University Hospital Trust and Wrightington Hospital in the North West region. A full medical history was taken for each patient using a proforma. Both hands and feet of each individual were examined. All cases had confirmed diagnosis of DD pre-operatively with the presence of characteristic Dupuytren's nodules in the palm of the hand and/or digits with contracture of either the metacarpophalangeal joint (MCPJ) or the proximalphalangeal joint (PIPJ).

**Controls:** Control individuals (n=20) were all age, sex and ethnically matched healthy Caucasian men and women selected from the same region of northwest of England. A full medical history was also taken using a proforma. Any scars and both hands of each individual were examined to rule out the presence of any skin fibrotic disorders. None of the controls had any evidence ofDD and reported no family history of DD.

The local and hospital ethical committees had given approval for the study. Written consent was obtained from all individuals.

### 1.1.2 (b) DNA Extraction

Blood samples were collected from subjects using a standard venesection technique. 15 mls of venous blood was collected from every subject. DNA was extracted from peripheral blood cells using a commercially available DNA extraction kit (Qiagen,UK). DNA concentrations were measured and diluted in buffer to 100 ng/µl using sterile, Qiagen buffer.

### 1.1.2 (c) Primer design

Eighteen sets of primers were designed in order to amplify the whole mitochondrial genome. Details of the mitochondrial fragments that they amplify are given in Table 3 and primer sequences shown in Table 4.

**Table 3: Primer, restriction digest and DHPLC details employed for scanning of the mitochondrial genome.**

| Fragment No | Mitochondrial Position | Fragment size after digestion (bp) | Restriction enzyme | DHPLC Temperatures (°C) |
|---|---|---|---|---|
| 1 | 15974-16409 | 436 | None | 58 |
| 2 | 16341-10 | 331 | None | 60 |
| 3 | 29-480 | 452 | None | 57,59 |
| 4 | 368-1713 | 211,276,372,487 | MboI | 57,58 |
| 5 | 1650-2841 | 273, 394, 525 | HaeIII | 56,59 |
| 6 | 2415-3811 | 125, 210, 278, 342, 442 | DdeI | 57,58,59 |
| 7 | 3429-4428 | 109,179, 242, 470 | HaeIII | 57,58,60 |
| 8 | 4180-5488 | 135, 247, 396, 531 | MspI | 55,56,57 |
| 9 | 5347-6382 | 122, 190, 233, 491 | HaeIII | 58,59 |
| 10 | 6318-7707 | 117, 162, 253, 354, 504 | MspI | 55,56,57,58 |
| 11 | 7644-8784 | 141,181,212,243,364 | HaeIII | 56,59 |
| 12 | 8643-9458 | 187,239,390 | DdeI | 57,59 |
| 13 | 9397-11397 | 248,312,366,487,588 | AluI | 54,57 |
| 14 | 11322-12852 | 178, 366, 435, 552 | HaeIII + MspI | 54,56,58 |
| 15 | 12753-13264 | 512 | None | 59 |
| 16 | 13172-14610 | 129, 177, 289, 382, 462 | AluI + DdeI | 55,57,59 |
| 17 | 14427-15590 | 191,235,297,441 | MboI | 57,59 |
| 18 | 15424-16451 | 218,353,457 | AluI | 55,57 |

**Table 4: Primer sequences for amplification of the mitochondrial genome**

| **Primers** | **SEQ ID No.** | **Primers** | **SEQ ID No.** |
|---|---|---|---|
| MT #1 F: ctc cac cat tag cac cca aag c | 13 | MT #10 F: ctg gag cct ccg tag ace taa c | 29 |
| MT #1 R: gag gat ggt ggt caa ggg acc | 14 | MT#10R: ggc ata cag gac tag gaa gca g | 30 |
| MT #2 F: tac agt caa atc cct tct cgt cc | 15 | MT #11 F: tat cac ctt tca tga tca cgc cc | 31 |
| MT #2 R: tcc agc gtc tcg caa tgc tat c | 16 | MT#11R.1: gtc cga gga ggt tag ttg tgg c | 32 |
| MT#3F: ctc acg gga gct ctc cat gca t | 17 | MT#12F: aac cga cta atc acc acc caa ca | 33 |
| MT #3 R: att agt agt atg gga gtg gga gg | 18 | MT#12R: gga tta tcc cgt atc gaa ggc c | 34 |
| MT #4 F: acc cta aca cca gcc taa cca g | 19 | MT#13F: aag cac ata cca agg cca cca c | 35 |
| MT #4.1 R: ttg tct ggt agt aag gtg gag tg | 20 | MT#13.1R: gtg gag tcc gta aag agg tat c | 36 |
| MT #5 F: aac tta act tga ccg ctc tga gc | 21 | MT#14F: ctc ctg agc caa caa ctt aat atg | 37 |
| MT #5 R: agg ttg ggt tct gct ccg agg | 22 | MT#14R: gga ttg ctt gaa tgg ctg ctg tg | 38 |
| MT#6F: ctc act gtc aac cca aca cag g | 9 | MT #15 F: ctg ttc atc ggc tga gag ggc | 39 |
| MT #6 R: tgt gtt gtg ata agg gtg gag ag | 10 | MT #15 R: agt tga ctt gaa gtg gag aag gc | 40 |
| MT#7F: ccc tac ggg cta cta caa ccc | 23 | MT#16F: ctt agg cgc tat cac cac tct g | 41 |
| MT #7 R: ccc gat agc tta ttt agc tga cc | 24 | MT#16R: taa gcc ttc tcc tat tta tgg gg | 42 |
| MT#8F: act tcc tac cac tca ccc tag c | 25 | MT#17F: cca tgc ctc agg ata ctc ctc a | 43 |
| MT#8R: gga gat agg tag gag tag cgt g | 26 | MT#17R: cgg aga att gtg tag gcg aat ag | 44 |
| MT#9F: cct acg cct aat cta ctc cac c | 27 | MT#18F: aaa gac gcc ctc ggc tta ctt c | 45 |
| MT#9R: ccc taa gat aga gga gac acc tg | 28 | MT#18R: agc gag gag agt agc act ctt g | 46 |

Four primer sets produced fragments that could be immediately analysed by simple DHPLC whereas the remaining 14 primers amplified products in the 800-2000b fragment size which were used in a multiplex DHPLC analysis after subsequent restriction digestion. Forward and reverse primers were combined for each fragment set at 5µM concentration for each primer.

Sequencing primers used for mutation verification were either taken from the original amplification primer set detailed above, designed using the Primer 3 program (http://www.genome.wi.mit.edu/cgi-bin/primer/primer3 www.cgipr) or from the literature (Levin et al 1999).

### 1.1.2 (d) PCR procedure

PCR reactions were performed in a 100µl volume containing approximately 100ng genomic DNA as template, 200µM each dNTP (Cruachem Ltd), 30pmol of each forward and reverse primer, 2.5 units of Optimase DNA polymerase and 10x Optimase reaction buffer containing 1.5mM MgSO₄ (Transgenomic Ltd). Amplification took place using an MJ research Tetrad Thermal cycler with cycling conditions outlined in Table 5. A small amount (5µl) of PCR product was tested on a 2% agarose gel stained with ethidium bromide and visualized under ultraviolet light.

**Table 5- PCR cycling conditions.**

| **Step** | **Temperature** | **Time** |
|---|---|---|
| 1 | 95°C | 3 minutes |
| 2 | 95°C | 30seconds |
| 3 | 63 -0.5°C per cycle | 1minute |
| 4 | 72°C | 1minute per 500bp |
| 5 | Go to step 2, 14 more times | |
| 6 | 95°C | 30seconds |
| 7 | 56°C | 1 minute |
| 8 | 72°C | 1 minute per 500bp |
| 9 | Go to step 6, 19 more times | |
| 10 | 72°C | 10min |
| 11 | 4°C | Hold |

### 1.1.2 (e) Restriction enzyme digestion

Restriction enzymes (Alu1, Dde1, Hae III, Mbo 1 and Msp1 - New England Biolabs) were used to digest the various PCR fragments as indicated in table 3. 88.5µl of PCR product was mixed with 10µl of the relevant restriction enzyme buffer and 1.5µl of restriction enzyme. Samples were incubated at 37°C for 2 hours and a small proportion (10µl) was tested on a 2% agarose gel stained with ethidium bromide. Samples containing an aberrant restriction pattern were identified. This change in fragment size created by a change in restriction pattern could lead to an alteration to the DHPLC analysis temperatures predicted. The fact that the mitochondrial genome has a relatively constant composition and that very few restriction site mutants were identified did not lead us to change the analysis temperatures to those shown in table 3.

### 1.1.2 (f) Heteroduplex formation

Prior to DHPLC analysis all samples underwent a heteroduplex formation step consisting of heating at 95°C for 5 minutes and then cooling at a rate of 1.5°C per minute until a temperature of 25°C was reached using the MJ Tetrad thermal cycling instrument.

### 1.1.2 (g) DHPLC Analysis

Sample analysis using the temperatures predicted (WAVEMAKER^{™} software Transgenomic Inc, NE, USA) shown in Table 3 was accomplished using the WAVE Nucleic Acid Fragment Analysis System and DNASep Cartridge technology (Transgenomic Inc, NE, USA). The gradient mobile phase consisted of Buffer A (0.1M Tri Ethyl Ammonium Acetate pH 7.0) and Buffer B (0.1M Tri Ethyl Ammonium Acetate pH 7.0 and 25%Acetonitrile). These eluents were mixed to produce a linear gradient varying Buffer B between 45 and 67% over a 12-minute period. The recommended cartridge cleaning procedures for this application were adopted; a 0.5minute clean using 75% Acetonitrile at the end of the linear gradient. Routinely 10-15µl of sample was injected per analysis.

### 1.1.2 (h) Sequencing

PCR for mutation verification by sequencing was carried out as outlined above using the relevant primer set. The samples were ExoSapIT treated prior to ethanol precipitation. Cycle sequencing, according to manufacturers protocols was performed using Applied Biosystems 3100 Sequencer.

### 1.2 RESULTS

### 1.2.1 HYPERVARIABLE REGION

Three primer sets (MT1-3) amplified the majority of the hypervariable region (D-loop) of the mitochondrial genome. This region is non-coding and spans 15887 to 648 bp of the mitochondrial genome. It is characteristically highly polymorphic.

### 1.2.1(a) Primer 1

This primer set amplified the region 15974-16409bp, yielding a 436 bp fragment. Multiple and non-specific DHPLC pattern variations were detected throughout the affected and control samples. These variations represented a large number of random mutations. There was no single dominant pattern present in any of the samples. This indicated that there were no obvious disease causing mutations. These findings are mirrored by the sequencing results (summarised in Table 6). DD11, CW1, CW4 (WT) were sequenced as representative samples.

### 1.2.1(b) Primer 2

This primer set amplified the region 16341-102bp, yielding a 331bp fragment.

Somewhat surprisingly, in the light of the hypervariable nature of this region, the DHPLC analysis yielded no obvious changes between the affected and the control population. No samples were submitted for sequencing.

### 1.2.1(c) Primer 3

Primer set 3 amplified the region 29-480bp, yielding a 452bp fragment. The results from this 452bp fragment were similar to those obtained with Primer set 1. The DHPLC results showed a high degree of pattern variability that confirmed the highly polymorphic nature of this region. This indicated that there were no obvious disease causing mutations because there was no single dominant pattern present in any of the samples. The sequencing data is summarised in table 6.

### 1.2.2 CODING REGION

### 1.2.2 (a) Primer 4

This primer set covers the region 368-1713bp (encoding 12srRNA at 648-1601bp) yielding a 1346bp product that was digested with Mbo1 to yield fragments of 211, 276, 372 and 487bp. Primer design necessitated a degree of overlap to ensure full coverage of the mitochondrial region and thus the beginning of primer 4 covers 300 bp of the terminal portion of the D-loop region.

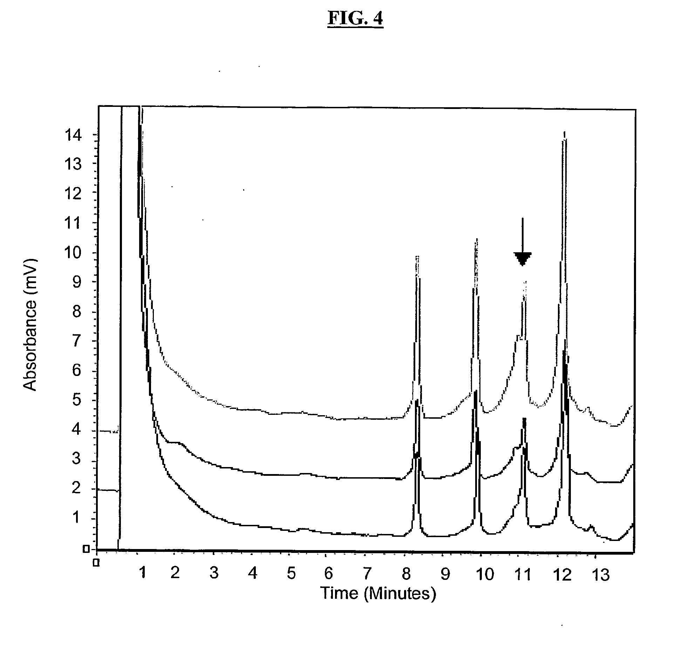

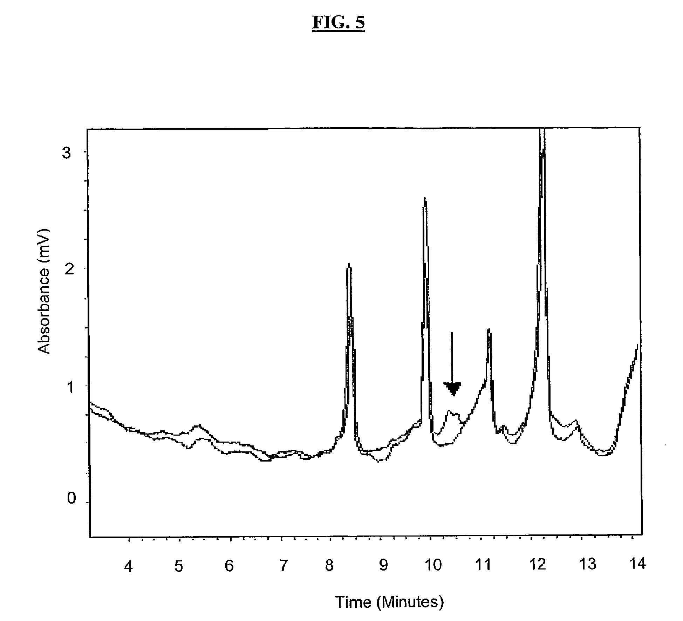

The known mutation DEAF is present in this region at position 1555. In the samples analysed no restriction site mutants were observed but DHPLC mutants were identified as shown in Figure 4 and 5 (see the arrows). Five samples were submitted for sequencing; DD1, DD7 and DD12 DHPLC mutants in fragment 3 and CW5 having a potential insertion in fragment 2. CW3 was sequenced as a control for both these regions. Mutations identified in these samples are summarised in Table 6.

### 1.2.2 (b) Primer 5

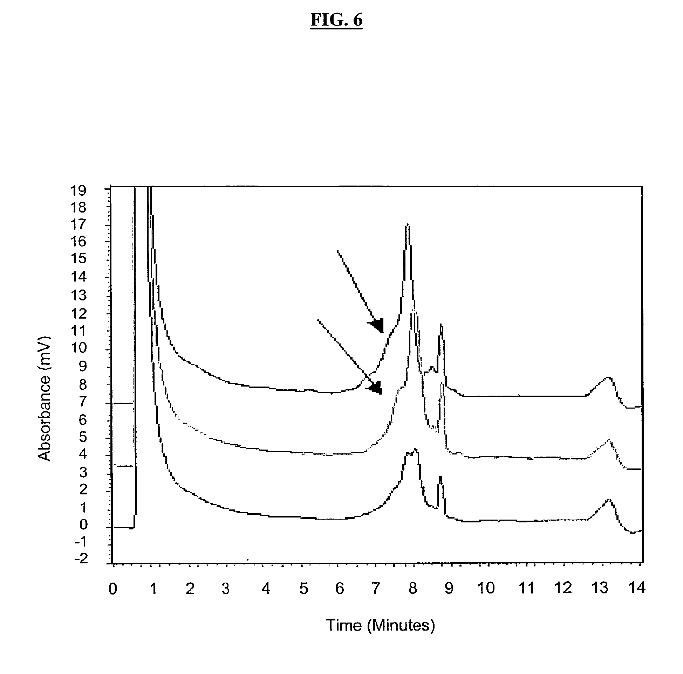

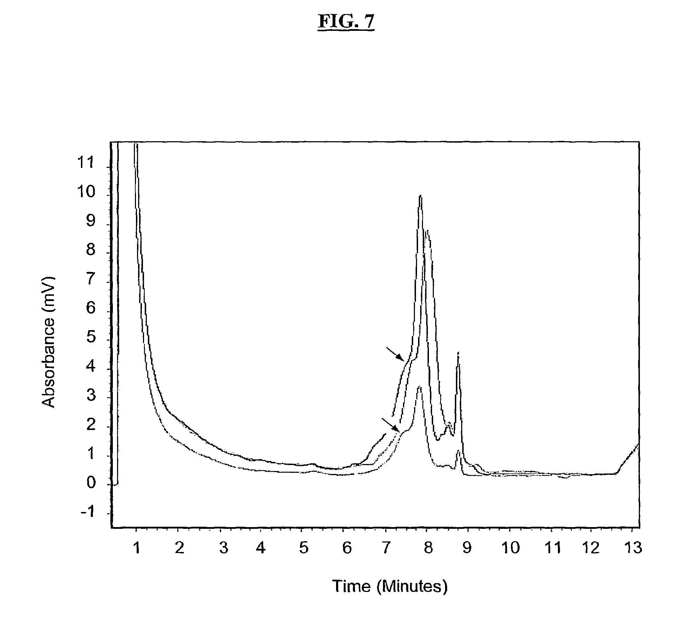

This primer set covers the region 1650-2841bp yielding a 1192 bp product that was digested with Hae III to yield fragments of 273, 394 and 525bp. No restriction site mutants were observed. DHPLC mutants were identified as shown in Figure 6. The degree of melting made it difficult to identify the precise fragment containing the mutant and therefore sequencing of fragment two and three was performed. Three samples DD 1, 6 and CW3 were submitted as being representative of DHPLC pattern changes with DD7 as reference pattern in this region (Fig. 6). The sequenced mutation at position 1690 in sample DD1 had a similar DHPLC pattern as in CW19, which is therefore likely to contain this unknown mutation (Table 6 and Fig 7). The known mutation in sample CW 3 at position 1811 bp (A>G) shares its DHPLC pattern with 6 other samples (CW4, 10 and 12; DD 6,12,20)

### 1.2.2 (c) Primer 6

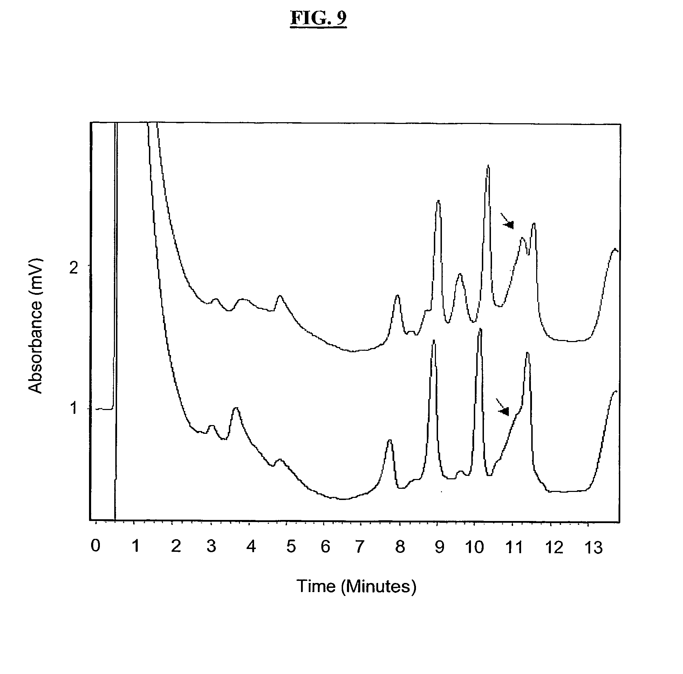

This primer set covers the region 2415-3811bp yielding a 1397bp product that was digested with Dde1 to yield fragments of 125, 210, 278, 342 and 442bp. DHPLC analysis showed a major change between the control (CW) and affected population (DD) in fragment 5 as illustrated in Figure 9. In DD samples there was a much more pronounced two-peak pattern in this fragment compared to the control samples that showed a major peak and shoulder. Four samples were submitted as representative for sequencing that is DD3 and DD11 with the controls being CW3 and CW5.

Out of 20 cases there were 2 PCR failures. 16 out of the 18 DD samples had a mutant DHPLC pattern (G>GT at position 2839) in comparison to the reference pattern in all the controls. None of the controls had the mutant pattern. This represents a significant correlation between the GT mutant and the incidence of DD. Accordingly a preferred method according to the invention detects for a mutation at position 2839 of the mitochondrial genome.

Another mutation was identified in one of the control samples CW3 at position T2706C. This was not present in any of the case and control samples sequenced.

### 1.2.2 (d) Primer 7

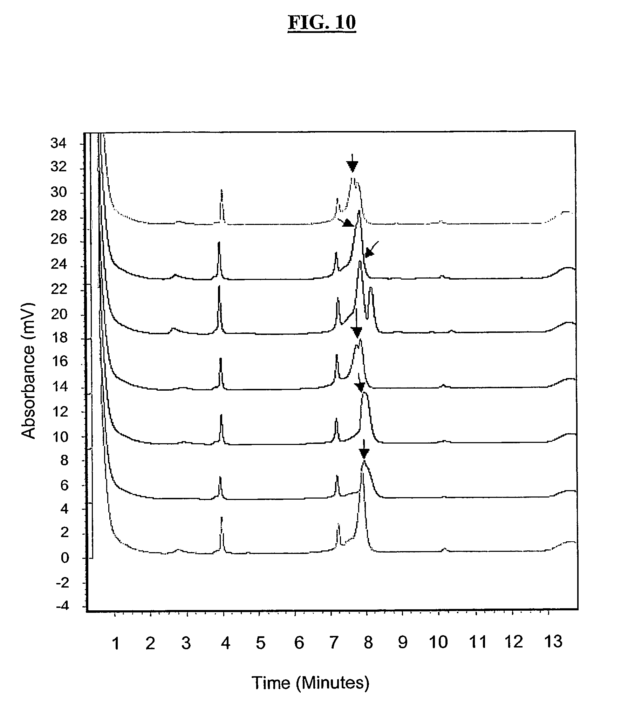

This primer set covers the region 3429-4428 yielding a 1000bp product that was digested with HaeIII to yield fragments of 109, 179, 242 and 470bp. In the samples there were no restriction site mutants present. DHPLC analysis showed a potentially interesting change in seven out of 20 affected samples as illustrated in Figure 10. The change is likely to be located somewhere in fragments 2, 3 or 4. All positive samples (DD1, 2, 5,11,14,17,18) were sent for sequence analysis with CW1, 9 and 15 as control samples (see Table 6 for mutations).

DD1, 2,11,14,17,18 all showed a different DHPLC pattern to the reference pattern (Fig 10). DD 1 and 14 are the same pattern, however DD11 is different to all patterns in this group.

### 1.2.2 (e) Primer 8

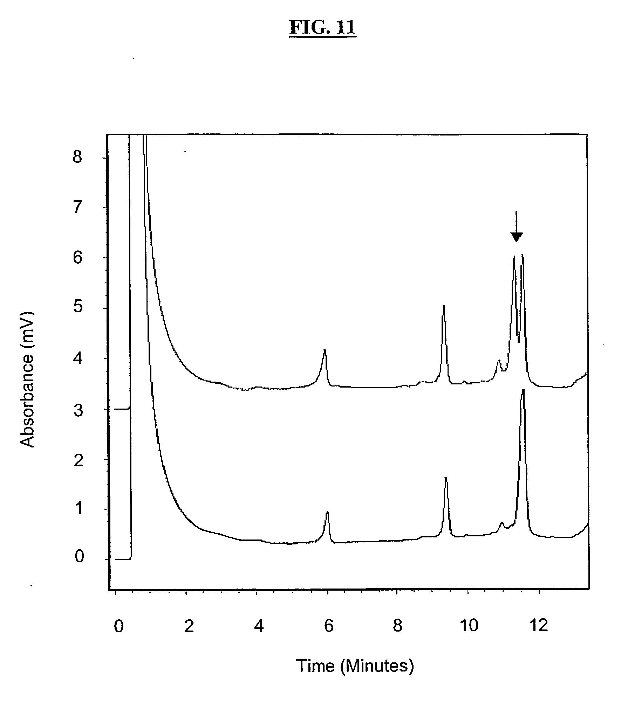

This primer set covers the region 4180-5488bp yielding a 1309bp product that was digested with MspI to yield fragments 135, 247, 396 and 531bp. There are no known reported disease mutations in this region. There was no restriction site mutant present in the analysed sample. DHPLC analysis showed a potentially interesting change in 94% of the cases that had a two peak pattern in comparison to 47% of controls (Figure 11). Three positive samples (DD10, 17 and 18) were sent for sequence analysis with CW2 as a reference sample (see Table 6).

The inventors believe the area 4180-4526 may have a mutation that will be relevant to conditions such as DD as 2 out of 3 DD samples had changes within this area.

### 1.2.2 (f) Primer 9

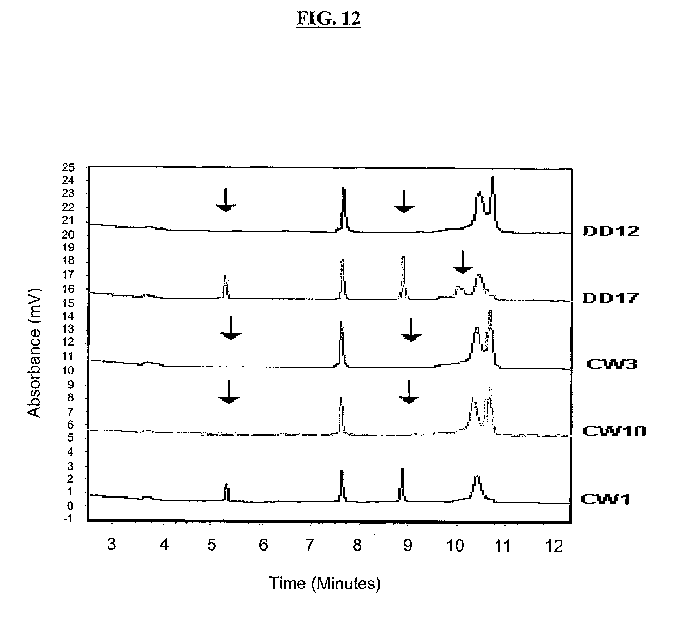

This primer set covers the region 5347-6382bp yielding a 1036bp product that was digested with Hae III to yield fragments 122, 190, 233 and 491bp. There are no known disease mutations in this region. Three samples DD12, CW3 and CW10 showed the same restriction site mutation that destroyed the Hae III cleavage site between 5836-5839bp (known). This restriction site mutation and an example of the DHPLC mutants (present in Fragment 4) can be seen in Figure 12.

### 1.2.2 (g) Primer 10

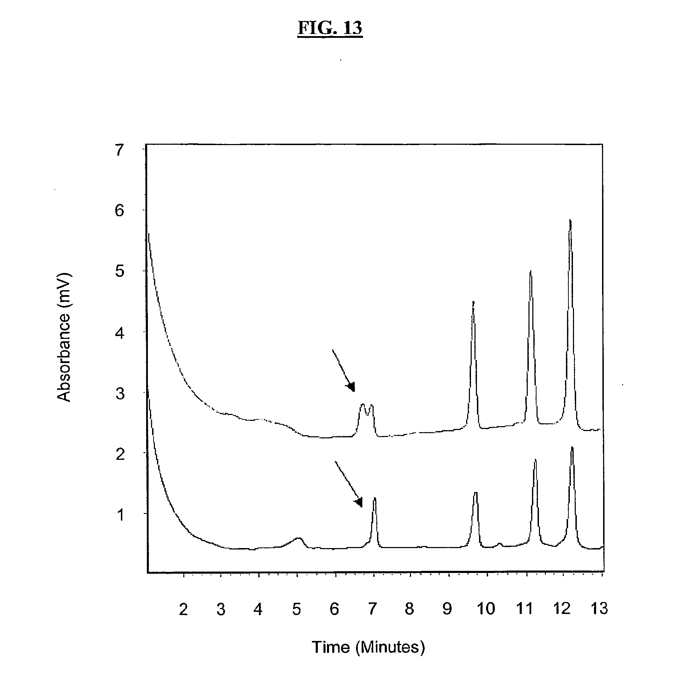

This primer set covers the region 6318-7707 yielding a 1390bp product that was digested with MspI to yield fragments 117, 162, 253, 354 and 504bp. In fragment 2, 100% of DD samples have a one-peak pattern whereas only 27% of controls have the one peak pattern. The DHPLC pattern change is illustrated in Figure 13. Seven samples were submitted for sequencing, 3 DD cases (DD1, 12 and 20) and 4 controls (CW1, 3, 5,and 7). However, sequencing revealed no changes in fragment 2 sequence of primer 10.

### 1.2.2 (h) Primer 11

This primer set covers the region 7644-8784 yielding a 1141bp product that was digested with HaeIII to yield fragments 141, 181, 212, 243 and 364bp. There are two known different restriction site mutations found in our samples that destroy Hae III sites.

Samples DD10 and CW14 have the restriction site destroyed at 8250 bp (G>A). Another restriction site mutation at position 7980 (A>G) was found in sample DD12. There is a 9 bp deletion (caccccctc) in samples DD17 and CW10 at position 8269-8277. Two restriction site mutants and the 9-bp deletion were confirmed by sequencing.

### 1.2.2 (i) Primer 12

This primer set covers the region 8643-9458bp yielding a 816bp product that was digested with DdeI to yield fragments of 187, 239 and 390bp. There are no restriction site mutants in the samples analysed.

We identified an unknown mutation present in one case and two controls at position 8696 (G>A). Another point mutation was also found in one control only at position 9264 (C>T). There were no other similar DHPLC pattern changes in our sample.

### 1.2.2 (j) Primer 13

This primer set covers the region 9397 - 11387bp yielding a 2001bp product that was digested with Alu1 to yield fragments of 248, 312, 366, 487 and 588bp. There are no known disease mutations. Two restriction site mutants DD17 and CW3 that destroy the Alu1 site 9643-9647bp (known) were identified.

### 1.2.2 (k) Primer 14

This primer set covers the region 11322- 12582bp yielding a 1531bp product that was digested with HaeIII and vlspI to yield fragments of 178, 366, 435 and 552bp. There is a known mutation for disease LHON (Leber's hereditary Optic neuropathy)(G11778A). There are no restriction site mutants in this area.

### 1.2.2 (l) Primer 15

This primer set covers the region 12753-13264bp yielding a 512 bp product. This was a simple DHPLC fragment that was analysed without restriction digestion at 59°C. There was no obvious change in the DHPLC pattern between affected and control populations.

### 1.2.2 (m) Primer 16

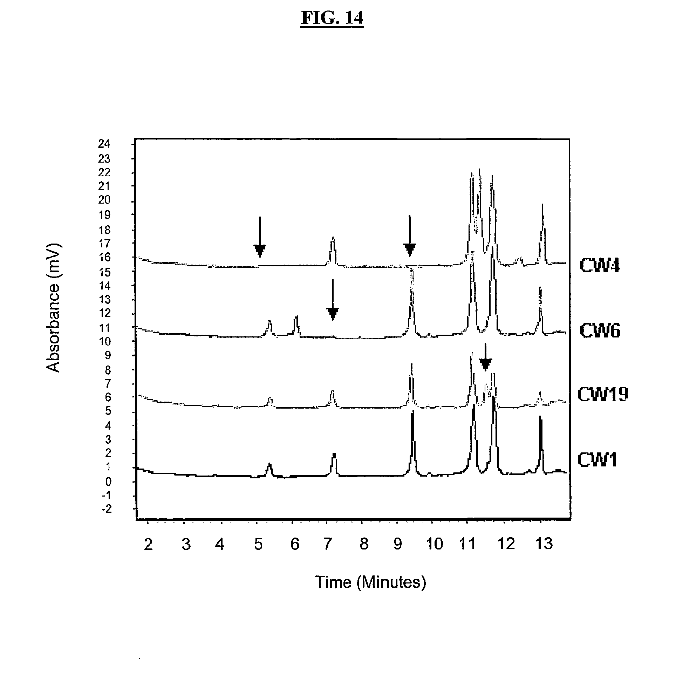

This primer set covers the region 13172 - 14610bp yielding a 1439bp product that was digested with AluI and Ddel to yield fragments 129, 177, 289, 382 and 462bp. There are two known disease mutations for LHON (Leber's hereditary Optic neuropathy)(A14495G and T14484C). There are three restriction site mutants in this area. The first two are known mutations; the first one is found in CW4, destroying the site Alu1 14014- 14017 and a second in Alu1 site at 14303-14306. The third restriction site mutant is unknown and is found in sample CW6 that destroys the Dde1 site 14433-14437) shown in Figure 14 with an aberrant DHPLC patterns in sample CW 19.

### 1.2.2 (n) Primer 17

This primer set covers the region 14427 - 15590 yielding a 1164bp product that was digested with MboI to yield fragments 191,235, 297, and 441bp. Three restriction site mutants were identified in DD12, CW13 and CW20 which destroyed the Mbo1 site (14868- 14871). Mutations identified in DD18 are shown in Table 6.

### 1.2.2 (o) Primer 18

This primer set covers the region 15424 - 16451 yielding a 1028bp product that was digested with Alu I to yield fragments of 218, 353 and 457bp. There are no known mutations but three restriction site mutants in DD17, CW9 and CW11 which create and Alu1 site in fragment 2 (15424-15776) splitting this into a 165 and 188bp product (AGCT) were identified.

Sequencing of DD17 identified mutations at 15606 (A>G) and 15451 (G>T).

Table 6 summarises mutations identified in Example 1 throughout the mitochondrial genome.

**Table 6:**

| **MT Set** | **MT Genome** | **bp-fragments** | **Relevant genes** | **Disease related mutations** | **Known mutations** | **Unknown mutations** |
|---|---|---|---|---|---|---|
| **MT #1** | 15974-16409 | 15974-16409 | D Loop | | DD11 G16361A; CW1 | DD11 A16355G; |
| | | | | | T16342C; | CW4A16355G; CW4 |
| | | | | | DD11A16182C; | A16223G; DD11 |
| | | | | | | T16188C; DD11 |
| | | | | | | Gl6158T/G DD11 |
| | | | | | | T16145C/G |
| | | | | | | DD11TI6148G; DD11 |
| | | | | | | A16151C; DD11 A |
| | | | | | | 16153C; DD11 |
| | | | | | | A16154C/A DD11 |
| | | | | | | C16155C/A DD11 |
| | | | | | | C16156C/A |
| **MT #2** | 16341-102 | 16341-102 | D Loop | | | |
| **MT #3** | 29-480 | 29-480 | D Loop | | DD10A73G; | CW19 G185A; CW12 |
| | | | | | CW12A73G; | T320C; |
| | | | | | CW14A73G; | |
| | | | | | CW19A73G; | |
| | | | | | DD10G143A; | |
| | | | | | CW12C150T; | |
| | | | | | CW12T152C; | |
| | | | | | CW13 T152C; | |
| | | | | | CW19A189G; | |
| | | | | | DD10T192C; | |
| | | | | | DD10C194T; | |
| | | | | | CW14C194T; | |
| | | | | | DD10T195C; | |
| | | | | | CW12T195C; | |
| | | | | | CW13T195C; | |
| | | | | | CW14T195C; | |
| | | | | | DD10T196C; | |
| | | | | | DD10T204C; | |
| | | | | | CW14T204C; | |
| | | | | | DD10G207A; | |
| | | | | | Cw14G207A; | |
| | | | | | CW12A215G; | |
| | | | | | CW19G228A; | |
| | | | | | CW12C295T; | |
| | | | | | CW19C295T; | |
| | | | | | CW12 309InsT; CW13 | |
| | | | | | 309InsC; CW14 309InsC; | |
| | | | | | | |
| **MT #4** | 368-1713 | F1 740-950 | 12s rRNA 648-1601 | | Cw19 C462T; | CW19 C489C/T; |
| | | | | | DD12 C479T; | |
| | | F2 951-1226 | | | | |
| | | F3 368-739 | | | | |
| | | F4 1227-1713 | | | | |
| **MT#5** | 1650-2841 | F1 2569-2841 | 16srRNA 1671-3307 | | CW3 A1811G | DD1 T1690C |
| | | F2 2175-2568 | | | | |
| | | F3 1650-2174 | | | | |
| **MT #6** | 2415-3811 | F1 3067-3191 | 16srRNA 1671-3307 | MELAS, CPEO | | DD3 G2839G/T; |
| | | | | A3243G | | DD11 G2839G/T; |
| | | | | | | CW3 T2706C |
| | | F2 2857-3066 | | MiMyCa A3260G | | |
| | | F3 3534-3811 | | MELAS T3271C | | |
| | | F4 3192-3533 | ND1 3307-4262 | LHON G3460A | | |
| | | F5 2415-2856 | | | | |
| **MT** #**7** | 3429-4428 | F1 3850-3958 | ND1 3307-4262 | | | DD5 C3991T; |
| | | | | | | DD14 C3991T; |
| | | | | | | DD5 G4023A; DD17 |
| | | | | | | T4215C; |
| | | | | | | Cw9 T 4215; |
| | | | | | | DD18 T4335C; CW15 |
| | | | | | | T4335C; DD14 T4002 |
| | | | | | | T/C DD18 T402T/C |
| | | | | | | DD14 A3996A/C |
| | | | | | | DD18 A3996A/C |
| | | | | | | DD14 G3981 G/T |
| | | | | | | DD18 G3981G/T |
| | | | | | | DD11 A3795G; CW1 |
| | | | | | | A3785A/G CW1 |
| | | | | | | G3593A |
| | | | | LHON G3460A | | |
| | | F23429-3607 | | LHON T4160C | | |
| | | F3 3608-3849 | | | | |
| | | F4 3959-4428 | | | | |
| **MT** #**8** | 4180-5488 | F1 4711-4845 | ND2 4470-5511 | | | DD17 A4916G; DD10 |
| | | | | | | G5045A; |
| | | F2 5242-5488 | | | | |
| | | F3 4846-5241 | | | | |
| | | F4 4180-4710 | | | | |
| **MT** #**9** | 5347-6382 | F1 6261-6382 | CO1 5904 - 7444 | | DD12 HaeIII -5837 | |
| | | F2 5838-6027 | | | CW13 HaeIII -5837 | |
| | | F3 6028-6260 | | | CW10 HaeIII -5837 | |
| | | F4 5347-5837 | | | | |
| **MT**#**10** | 6318-7707 | F1 6571-6687 | COI 5904 - 7444 | PPK A7445G | | |
| | | F2 6688-6849 | CoII 7586-8262 | | | |
| | | F3 6318-6570 | | | | |
| | | F4 6850-7203 | | | | |
| | | F5 7204-7707 | | | | |
| **MT** #**11** | 7644-8784 | F18251-8391 | ATPase8 8366-8527 | MERFF A8344G | | DD17 8269-8277 del |
| | | | | | | 9bp |
| | | | | | DD10 HaeIII-8250 | |
| | | F2 8392-8572 | ATPase6 8527-9207 | MERFF T8356G | CW14 HaeIII-8250 | |
| | | F3 8573-8784 | | | DD12 Hae III-7980 | |
| | | F4 7644-7886 | | | | |
| | | F5 7887-8250 | | | | |
| **MT**#**12** | 8643-9458 | F1 9272-9458 | | NARP T8993G | | DD17 G8698A; |
| | | | | | | CW9G8686A; |
| | | | | | | Cw11G8696A; CW1 |
| | | | | | | C9264T; |
| | | | ATPase6 8527-9207 | | | |
| | | F2 8643-8881 | CoIII 9207-9990 | | | |
| | | F3 8882-9271 | | | | |
| **MT** #**13** | 9397-11397 | F1 9397-9644 | | | | |
| | | | CoIII 9207-9990 | | DD17 Alul -9646 | |
| | | F2 10599-10910 | ND3 10059-10404 | | CW3 Alul -9646 | |
| | | F3 10233-10598 | ND4L 10470-10766 | | | |
| | | F4 10911-11397 | ND4 10760-12137 | | | |
| | | F5 9645-10232 | | | | |
| **MT** #**14** | 11322-12852 | F1 12123- | | LHON G11778A | | |
| | | 12300; F2 | | | | |
| | | 11322-11687 | ND4 10760-12137 | | | |
| | | | ND5 12337-14148 | | | |
| | | F3 11688-12122 | | | | |
| | | F4 12301-12852 | | | | |
| **MT** #**15** | 12753-13264 | 12753-13264 | | | | |
| **MT** #**16** | 13172-14610 | F1 14305- | ND6 14149-14673 | LHON T14484C | CW4 Alu1 -14015; CW4 | CW6 A14586G; CW19 |
| | | 14433; F2 | | LHON A14495G | Alu1 -14304; | A13220C; Cw6 Dde1 - |
| | | 14434-14610; | | | | 14433 |
| | | F3 14016- | | | | |
| | | 14304; F4 | | | | |
| | | 13172-13553; | | | | |
| | | F5 13554-14015 | | | | |
| **MT**#**17** | 14427-15590 | F1 14868- | Cytb 14747-15887 | LHON G15257A | DD12Mbol-14869; | DD18 15434insC; |
| | | 15058; F2 | | | Cw13Mbol-14869; | DD18 T15362T/C |
| | | 15356-15590; | | | Cw20Mbol-14869 | DD18T15332C/T |
| | | F3 15059- | | | | DD18G15336G/C |
| | | 15355; F4 | | | | DD18 T15339T/C |
| | | 14427-14867 | | | | DD18 G15319C/G |
| | | | | | | DD18 A15282C/A |
| | | | | | | DD18G15274C/G |
| | | | | | | |
| **MT**#**18** | 15424-16451 | F1 15777- | Cytb 14747-15887 | LHON G15257A | | DD17 A15606G; |
| | | 15994; F2 | | | | DD17A 15415G; |
| | | 15424-15776; | | | | |
| | | F3 15995- | | | | |
| | | 16451. | | | | |

### 1.3 DISCUSSION

### 1.3.1 Mutations

### 1.3.1 (a) D-Loop or hypervariable region (PRIMERS 1-4)

The presence of the hypervariable site in the non-coding region of human mtDNA has been well documented in the past. Hypervariable sites are thought to represent mutational hotspots as germline and somatic mutations preferentially occur at this site. This region was found to be a mutational hot spot in lung, bladder and head and neck cancers. Mutations in this region might be related to the function of the D-loop as a regulatory site for replication and expression of the mtDNA. The unique feature of these hypervariable sites to make them such mutational hotspots is not as yet known.

Primers 1 to 3 cover the majority of the D-loop region with the terminal 300bp being covered by primer 4. A number of shifts and pattern changes were observed that were present randomly throughout the DHPLC analysis for primer set 1 and 3. This is reflected in the sequencing data where a number of changes were seen in both control and case samples. Overall no disease specific mutations were found in these primer sets.

In primer 1, we found 10 unknown mutations in DD cases and 5 point and 5 heteroplasmic mutations with 2 unknown point mutations in controls. We also found two known point mutations in cases and one in a control.

There were no obvious DHPLC pattern changes in primer 2 and therefore no samples were submitted for sequencing even though, in the mitochondrial polymorphism reference database (http://infinity.gen.emory.edu/mitomap.html) there are twenty plus mutations reported in this region. DHPLC pattern comparison, for example between primer 1 and 2 shows significantly decreased variability in primer 2 analysis and in particular no major pattern changes or shifts were identified. In addition, these reported changes may not actually be present within our sample population.

In primer 3 there two unknown point mutations were identified in the controls. We identified two known and one unknown mutations in primer 4, the positions of which indicated that they belong to the D-loop region. Only two out of twenty cases demonstrated this DHPLC pattern, which was absent from all controls. This was confirmed be sequencing. In conclusion there is not a strong correlation between D-loop polymorphisms and DD.

### 1.3.1 (b) Ribosomal RNA - rRNA 12s,16s (PRIMERS 4-6)

Primer 4 covers completely the 12srRNA. Primer 5 covers the majority of 16srRNA with the beginning and end of the 16srRNA being covered by primers 4 and 6 respectively. We have found no mutations in the 12srRNA region. This is not a surprising finding in view of the fact that this region is not such a mutational hotspot as the hypervariable region and the incidence of mutations should be lower. There are however reported disease mutations in the 12srRNA region which include the following examples; SNHL (1095C), DM (1310T, 1438T) and DEAF (1555G).

An already know mutation was identified at position 1811 bp in the 16sRNA, which was present in 3 cases and 4 controls. In addition, an unknown mutation was identified at position 1690 in one case with a similar DHPLC pattern in a control - likely to be the same sequence. The distribution of both these mutations (1690 and 1811 bp) between cases and controls does not appear to show any significance to DD.

An unknown heteroplasmic mutation at position 2839 bp which was a G to G/T base pair change in the 16srRNA region of the mitochondrion was identified. The DHPLC pattern change was clearly evident in approximately 90 % of the cases and absent from all controls. As indicated above, this mutation demonstrated a significant linkage with DD. Accordingly this mutation represents a most preferred mutation which may be tested according to the method of the invention.

A novel mutation was also identified in a single control, which was not present in any of the other samples that were sequenced. The high prevalence of the DHPLC pattern change and its marked differentiation between cases and controls may potentially indicate disease relevance. A heteroplasmic mutation in the 16srRNA is presumed to alter mitochondrial protein synthesis.

Other known disease mutations have been described in the 16srRNA region. These include the following, Rett syndrome (2835T), MELAS (3093G) and ADPD (3196A).

### 1.3.1 (c) tRNA

We found three samples (one DD and two controls) where the Hae III restriction site at 5837 bp had been destroyed resulting in a different fragment distribution. The cut sequence was GGCC. The position of this mutation falls within the tRNA coding region between ND2 and COI. Searching the Mitochondrial DNA Base Substitution Diseases for rRNA and tRNA mutations in the mitomap database (http://www.gen.emory.edu/cgi-bin/MITOMAP/bin/tb19gen.pl), it became evident that the mutation at position 5837 has not been previously reported. This mutation is at a relatively low frequency in our samples and is present in both our cases and controls therefore this does not appear to be of relevance to DD.

An unknown 9 bp deletion was found at position 8260-8277, which maps to tRNA (K) in one case and one control. Interestingly, a 9 bp deletion at position 8271-8281 has been reported previously (http://www.gen.emory.edu/cgi-bin/MITOMAP/bin/tb110gen.pl); however, this reported deletion is not in exactly in the same location but overlaps the one we have found. There is a published mutation within this region (A7445G) associated with Palmoplantar keratoderma (PPK). PPK affects the palms and soles and is presumed to be a heterogeneous genetic skin disorder.

### 1.3.1 (d) OXPHOS Polypeptide subunits

### COMPLEX I: (PRIMER 7,8,13,14,15,16)

### NADH dehydrogenase (ubiquinone) chain 1 -6 (ND1, 2, 3, 4, 4L, 5 and 6)

We found a striking DHPLC pattern in the ND2 region in 94 % of cases that was only present in 45 % of controls. We also found two novel mutations at positions A4916G and G5045A in two cases that are also present in the ND2 region.

The NADH-quinone oxidoreductase (complex I) is one of three energy-transducing enzyme complexes of the respiratory chain in mitochondria. It is the point of entry for the major fraction of electrons that traverse the respiratory chain eventually resulting in the reduction of oxygen. Complex I is presumed to be one of the most intricate membrane-bound enzymes known to date, being composed of at least 43 unlike polypeptides. Of these, 7 are encoded by mtDNA.

An Alu I restriction site mutant at position 14015 was found in one control. This is a known mutation in the ND5 region. There was also another point mutation A13220C in a control sample found in ND5. None of our mutations in ND 5 region were in the affected population therefore presumed not to be disease relevant.

In the ND6 region we identified three different mutations, which were present in the control samples only. A known Alu I restriction site mutant at position 14303 and a novel DdeI restriction site mutant at 14433 in another control sample was found. A single base pair mutation was identified at position A14586G in a control sample. As there were no mutations present in our affected samples, the ND6 region is presumed not to be related to DD.

A large number of human mitochondrial diseases involve structural and functional defects at the level of enzyme complex I. A variety of neuromuscular diseases, including myoclonic epilepsy and ragged red fibers (MERRF); mitochondrial encephalomyopathy, lactic acidosis and stroke-like episodes (MELAS); Chronic External Ophthalmoplegia Plus (CEOP); Kearns-Sayre syndrome (KSS); and cases of Leber's hereditary optic neuropathy (LHON) appear to be associated with a defect in complex I. The defect identified in most of such cases is a point mutation in the mtDNA.

### COMPLEX II: Succinate dehydrogenase complex

This complex is coded by nuclear DNA and therefore not scanned as part of the whole mtDNA screening.

### COMPLEX III: Cytb (Primers P17,18)

Cytochrome b-c1 complex ubiquinol--cytochrome-c reductase cytochrome b - mitochondrion

We identified multiple novel mutations in two cases that showed a very similar DHPLC pattern. These sequence changes were not present in any of our controls. The majority of changes found by sequencing revealed the novel presence of heteroplasmy which included G15274C/G; A15282C/A; G15319C/G; T15332C/T; G15336G/C; T15339T/C; T15362C/T and 15434insC. All these changes were in Cyt b region. A known restriction site mutant was also identified in one case and two controls at position MboI 14869. Although the multiple unknown heteroplasmic mutations identified in our cases are present at a low frequency, they are absent from our controls and may therefore have some relevance to the disease. The presence of the restriction mutant is not disease specific as it is more frequent in the control population.

### COMPLEX IV: Cytochrome-c oxidase chains I -III (CO I, II, III) (Primers 9,10,11,12,13)

We found prominent DHPLC pattern change in our samples within 6688 and 6849 the COI region. All cases had the wild type single peak pattern, whereas only 27 % of the control samples had a similar pattern. It is surprising that sequencing had not yielded an obvious base pair substitution within this region. One hypothesis would be that the mutation is at too low a level to be identified by sequencing.

Two restriction mutations were confirmed by sequencing that localised to the COII region. The restriction mutation at position 8250 is present in one case and one control only. There is a single restriction mutation at position 7980 found in one case. The frequency and distribution of the mutations in this region are not sufficient to be disease associated.

### COMPLEX V: ATPase Synthase proteins 6 & 8 (Primers P11, 12)

A DHPLC pattern change was identified in one case and two controls at position G8696A that was confirmed by sequencing. This was located in the ATPase 6 region of the mitochondrial genome. There is a known disease mutation NARP (Neurogenic weakness, ataxia and retinitis pigmentosa (T8993G) in this region. We assume that the mutation we found is not DD related because of its low prevalence amongst the cases.

A wide variety of conditions have been linked to mutations in mitochondrial genes. Some of these genes are located either in nuclear DNA (nDNA) or the mtDNA. The inheritance patterns in mitochondrial disorders can also range from maternal, Mendelian and a combination of the two. We have solely investigated the mtDNA in maternally inherited pedigrees for the presence of mutations in relation to our cases and controls. The identification of novel polymorphism in the 16srRNA region that is present in more than 90% of the cases and absent from controls is an important finding. It is possible that this mutation is pathogenic and may cause an oxidative phosphorylation defect by interfering with mitochondrial protein synthesis.

### 1.3. 2 Technology

The original technique for identification of mutation in the mitochondrial genome using a multiplex DHPLC technique (Van den Bosch *et al*., 2000) approached the issue of mutation detection from a diagnostic standpoint; to scan a few samples in such a way that the possibility of missing any diagnostic mutations would be negligible. Although this is the ideal outcome for any mutation detection project this ideal of finding all mutations is not always realistic when scanning a large genomic area with multiple samples. However, DHPLC is the best approach for undertaking such scanning projects as very little compromise needs to be made for sample size, due to the automation of the instrumentation, or for accuracy, due to its ability to find mutations in a non-position dependent manner. Blind analysis papers repeatedly ascribe 95-100% efficiency at finding mutations using DHPLC. Importantly with regards to detecting the presence of heteroplasmic mutations its ability to detect even the presence of small quantities of heteroduplex indicating a mutation has also been reported.

DHPLC detection of mutations is dependent on the polymerase chain reaction (PCR) to amplify human genomic DNA for analysis. Like all enzymes, Taq DNA polymerases have an inherent error rate of approximately 8.0x10⁻⁶ to 2x10⁻⁴. These misincorporations by the Taq polymerase are left uncorrected and are then copied in subsequent rounds of amplification. Alternatively, proof reading polymerase enzymes (Pfu and Pwo) have a lower error rate of approximately 1.3x10⁻⁶. Each error introduced at the amplification can be considered to be equivalent to a random low-level mutation. Within normal mutation detection systems these errors, although replicated in future rounds of PCR amplification, will be at a considerably lower level than a true heterozygote mutation. For this reason fidelity of the PCR enzyme may not be a major issue for routine mutation detection except in extreme cases of low template level and excessive PCR cycling conditions. However when detecting mutations in DNA populations where there is not an equal proportion of wild type and mutant alleles (tumour sample or mitochondrial detection for example) the fidelity of the enzyme will play a more prominent role. In extreme cases the levels of mutation and errors of misincorporations by the PCR enzyme may be comparable.

The misincorporations by the Taq enzyme is viewed on a DHPLC chromatogram, at the analysis temperature of the fragment, as a characteristic shoulder to the main peak. DHPLC calls mutations as changes to a reference pattern - a positive mutation call is not always a defined peak pattern. These two latter facts taken in conjunction may mean that very small changes in pattern can occur which could be masked by the misincorporations peak preceding the main reference peak. For this reason, in particular when scanning the mitochondrial genome, we have chosen to use a novel proof reading polymerase (Optimase^{™} DNA polymerase, Transgenomic Inc, NE, USA) which has been shown to have the lowest misincorporation level in comparison to other proof reading and Taq based polymerases commercially available. Figure Y5 illustrates this comparison by calculating the percentage heteroduplexes formed using a number of commercially available enzymes.

As a substrate for the PCR amplification, genomic DNA has been isolated from the blood of 40 case and age, sex matched control samples. The acquisition of blood samples is relatively non invasive in comparison to dissection of tissue and in addition reasonable quantities of material can be obtained. Importantly however with regard to mitochondrial syndromes there can be tissue specific expression in relation to mitochondrial related disease. It is therefore possible that the highest levels of mutations may not be found in blood. Positively however pathogenic mtDNA mutations are likely to be heteroplasmic and for this reason it was not felt necessary to mix samples with a reference (wild type) sample in order to ensure the presence of a heterozygous condition and hence detection of mutations. In addition it would be impossible to find a true wild type by definition of the relatively high mutation rate within the mitochondrial genome should this be necessary.

We have chosen to amplify the mitochondrial genome with a total of 18 overlapping fragments using the Optimase polymerase. Four of these fragments are designed to undergo "normal" DHPLC ranging in size from 331 to 550bp. In particular three of these fragments span the hypervariable region; splitting these into individual PCR reactions will make DHPLC recognition and verification by this approach easier. The remaining fourteen PCR reactions covering the rest of the coding region of the genome undergo restriction digestion with a combination of 5 restriction enzymes to produce the multiplex DHPLC fragment combinations that are analysed at a range of temperatures. The mitochondrial genome has a relatively constant GC composition making the range of temperatures needed for such DHPLC scanning less than would be normally required for other genomes. It could also be said that DHPLC's accuracy at locating mutations can be largely attributed to its ability to find mutations over a range of temperatures. Of particular note in the original technique is the choice to repeatedly scan at one-degree intervals. We believe this level of scanning to be over cautious and may be acceptable for a diagnostic approach where certainty with regard to patient diagnosis is critical. The ability to detect mutations over at least 2-3°C if not at significantly higher windows of temperatures in conjunction with the redesign of the primer sets enables the choice of temperatures to be narrowed from 65 different analysis to 29 conditions to scan all fragments of the mitochondrial genome.

The key criteria to be considered when utilising DHPLC for mutation detection is to be confident in calling mutations as distinct changes in pattern. It has been shown that highest efficiencies at finding mutations are achieved when this pattern change calling is carried out at its simplest level. Importantly even small changes in peak shape can be correlated with the presence of a mutation. It is not strictly possible in a scanning context to locate a pattern change with a particular DNA base or position change as the patterns obtained have been shown to be dependent on base change, local and overall fragment sequence context. This could be argued to be even more critical with regard to locating mutations in mitochondrial populations due to the reasoning's already discussed. An added difficulty is mutation verification that normally occurs by sequencing. Sequencing will look at each base position and call the base that predominates at that position as being the correct sequence read. If it "sees" two bases at any one position it may call the base as N; subsequent checking of the electropheretogram will then determine the heterozygous nature of that position. Unfortunately when the bases at a position are not of equal magnitude then it may not denote N. Sequencing has been shown to have difficulties recognising heterozygosity at less than 30%. With regards to this research we therefore have a situation where we maybe able to detect mutations to an extremely low level but have difficulty verifying by sequencing. To an extent this has been circumvented by individual checking of the sequence reads to detect heteroplasmy; the proof of this being the identification of the heteroplasmic mutation in primer 6. At extreme cases other publications have shown that it has been necessary to clone potentially mutant DNA and then sequence large numbers of individual clones to verify potential mutants. An alternative strategy would be to collect the heteroduplex portion of the mutant DNA thereby enriching the sequencing population for mutant allele in the hope that the level was such as to be able to get the correct sequence call.

### 1.3.3 The relevance of the G2839G/T Mutant

There are thought to be four major criteria for supporting the deleterious role of a novel mutation. Firstly, the mutation must not be a neutral polymorphism; secondly the base change must affect an evolutionary conserved and functionally important region; thirdly deleterious mutations are usually heteroplasmic and fourthly the degree of heteroplasmy in different family members has to be in agreement with the severity of symptoms.

As regards to the first criterion, we have checked through the existing mitochondria databases and have not been able to locate the G2389G/T mutation. We therefore believe it represents a novel polymorphism. Although linkage with DD is clear, we are unsure of the functional importance of this particular polymorphism. A bioinformatic search is still underway to determine its functional and evolutionary significance. This particular polymorphism is heteroplasmic and is absent from our control population. The degree of heteroplasmy within our family pedigrees has not been determined, however, clinical examination of affected members of some of our pedigrees demonstrates a variety of phenotypic expression in DD.

Mitochondrial mutations leading to functional impairment of the respiratory chain are pathogenic. However, the genotype and phenotype relationship in mtDNA and affected disorders are difficult to understand. It is unclear why defects in the metabolic pathway that result in energy crisis can present with such range of symptoms and variety of tissues and organs. The same mtDNA mutation can produce very different phenotypes and different mutations can produce similar phenotypes. This may be due to the varying ratios of wild type to mutant mtDNA, the modulating effect of other mitochondrial and nuclear genes, and the different thresholds of biochemical expression for the tissue and the particular mutation involved.

The accumulation of base changes in mtDNA occurs over a short time scale to generate polymorphisms within populations of the same broad ethnic background, such as in Caucasians. There are mtDNA polymorphisms distinguishing the different human populations and the presence of heteroplasmic polymorphisms within an individual. However, most individuals are homoplasmic, which is presumed to arise from a severe bottleneck in oocyte development.

It has been proposed that mtDNAs with substantial mutations replicate more rapidly and therefore have a selective advantage over normal mtDNAs. This may account for the observation that high proportions of mutated mtDNA were found present in specific tissues such as muscle compared to other tissues like liver with lower proportion of mutations. The percentage of mutated mtDNA is presumed to increase with age in muscle. If the total number of muscle fibres does not change appreciably after birth therefore mtDNA replication is dissociated from cell division and there is positive selection for defective cells with mutated mtDNA. The variation in age of onset is thought to be due to the percentage of mtDNA present at birth and thereafter during birth, it is the rate of accumulation of mutated mtDNA compared to normal mtDNAs.

A number of interesting studies have provided evidence for future treatment of mitochondrial disease. PNAs are sequence specific peptide nucleic acids that are shown to bind selectively to mutant mtDNA and inhibit mtDNA replication. These agents may reduce the percentage of mutant mtDNA to sub-threshold level in the cell and as a result may lead to correction of the mitochondrial biochemical defect.

### 1.4 CONCLUSIONS

An enhanced multiplex Denaturing High Performance Liquid Chromatography (DHPLC) technique was employed to screen the whole mitochondrial genome in twenty cases of Dupuytren's disease with maternally transmitted inheritance pattern and twenty matched Caucasian controls.

Using this approach we confirmed a number of known mutations and more importantly several unknown mutations were identified. A proportion of these novel mutations were in the D-loop region and complex I, III and V in DD cases only.

Most interestingly, an unknown heteroplasmic mutation at position 2839 bp which was a G to G/T base pair change in the 16srRNA region was clearly evident in approximately 90 % of DD cases and absent from all controls. This mutation may affect mitochondrial protein synthesis in a tissue specific manner.

Our DHPLC technique is a robust and accurate method in predicting mitochondrial mutations and we were able to identify previously known and some novel disease related mutations. Understanding the detailed genetic basis of Dupuytren's disease is key to providing future prognostic and diagnostic advice to patients and to developing novel therapeutic regimes for treatment of this condition. This is the first report of a study in DD and mitochondrial mutations using DHPLC.

### EXAMPLE 2

Having established a correlation between a condition characterised by inappropriate scarring or fibrosis and a genotype conferred by the 2839 mtDNA polymorphism, the methodology of Example 1 was repeated to screen subjects according to the method of the first aspect of the invention to establish the genotype of the subj ect.

The results of the genetic test could then be used by a clinician to provide medical advice to individuals having the mutation. For instance, asymptomatic individuals found to have the mutant allele may be advised that they were at increased risk of developing a condition characterised by inappropriate scarring or fibrosis. If appropriate, such individuals could be advised to make life style changes and/or receive prophylactic treatment. Individuals having the 2839 mutation, who are already suffering from a condition characterised by inappropriate scarring or fibrosis, may be advised to adjust their medication or habits as they are potentially at risk of developing a more severe form of the condition.

## Claims

1. An *in vitro* method for diagnosing or detecting a predisposition to a condition at least partially **characterised by** inappropriate fibrosis or scarring, the method comprising examining the 16srRNA gene of the mitochondrial genome from a subject of interest to detect the presence of a genetic polymorphism or mutation linked to the development of the condition.

2. A method according to claim 1 wherein a region around position 2839 of the mitochondrial genome is examined to detect the presence of a genetic polymorphism or mutation.

3. A method according to claim 2 wherein an insertion of a thymidine nucleotide (T) after the guanosine nucleotide (G) (G2839G/T) at position 2839 of the mitochondrial genome is examined.

4. The method according to any one of claims 1 - 3 wherein the condition is Dupuytren's Disease.

5. The method according to any one of claims 1- 3 wherein the condition is a keloid or hypertrophic scar.

6. The method according to any one of claims 1 - 3 wherein the condition is any one of sclcroderma; systemic sclerosis; crest syndrome; tuberous sclerosis with skin patches; familial cutaneous collagenoma; metabolic and immunologic disorders of the skin; eosinophilic facsitis; discoid lupus erythematosus; dermatomyositis; mixed connective tissue disease; drug-induced skin fibrosis; oral submucous fibrosis; fibrosis induced following dietary and environmental exposures; pulmonary/cardiac fibrosis; liver fibrosis/cirrhosis; renal fibrosis; drug induced fibrosis; central and peripheral nervous system fibrosis; vascular system fibrosis; male and female genitourinary tract fibrosis; and gynaecological fibrosis.

7. The method according to any one of claims 1-3 wherein the condition is many one of cirrhosis of the liver, liver fibrosis, glomerulonephritis, pulmonary fibrosis, cystic fibrosis, scleroderma, myocardial fibrosis, fibrosis following myocardial infarction, and central nervous system fibrosis following a stroke or neuro-degenerative disorders

8. A method according to any preceding claim wherein the mitochondrial DNA is isolated from blood or tissue samples taken from the subject and then examined to detect the presence of a genetic polymorphism or mutation linked to the development of the condition.

9. A method according to any preceding claim wherein mitochondrial DNA is amplified before it is examined to detect the presence of a genetic polymorphism or mutation.

10. The method according to claim 9 wherein the mitochondrial DNA is amplified using the Polymerase Chain Reaction (PCR).

11. The method according to claim 10 wherein a pair of PCR primers of SEQ ID No 9 and 10 or a pair of primers of SEQ ID NO. 11 and 12 are utilised to amplify the mitochondrial DNA.

12. A method according to any preceding claim wherein the mitochondrial genome is examined by restriction digestion and size analysis.

13. A kit, for use in the method according to any one of claims 10-12, comprising:
A) PCR primers for amplifying genetic polymorphisms or mutations in the mitochondrial 16srRNA gene that are linked to a condition characterise by inappropriate fibrosis or scarring;
B) Control DNA samples of known genotype for each polymorphism or mutation; and
C) a data card outlining linkage between a particular polymorphism and a condition at least partially **characterised by** inappropriate fibrosis or scarring.

14. The kit according to claim 13 wherein the PCR primers are as defined in claim 11.

15. The kit according to claims 13 or 14 wherein the kit further comprises a suitable restriction enzyme for generating fragments of DNA.

## Patentansprüche

1. In-vitro-Verfahren zum Diagnostizieren oder Nachweisen einer Prädisposition für einen Zustand, der mindestens teilweise durch eine ungünstige Fibrose oder Vernarbung **gekennzeichnet** ist, wobei das Verfahren die Untersuchung des 16srRNA-Gens des mitochondrialen Genoms von einem Individuum von Interesse zum Nachweis der Anwesenheit eines genetischen Polymorphismus oder einer Mutation, der/die mit der Entwicklung des Zustands verknüpft ist, umfasst.

2. Verfahren nach Anspruch 1, worin eine Region um die Position 2839 des mitochondrialen Genoms herum zum Nachweis der Anwesenheit eines genetischen Polymorphismus oder einer Mutation untersucht wird.

3. Verfahren nach Anspruch 2, worin eine Insertion eines Thymidinnukleotids (T) nach dem Guanosinnukleotid (G) (G2839G/T) an der Position 2839 des mitochondrialen Genoms untersucht wird.

4. Verfahren nach einem der Ansprüche 1 bis 3, worin der Zustand die Dupuytren-Krankheit darstellt.

5. Verfahren nach einem der Ansprüche 1 bis 3, worin der Zustand ein Keloid oder eine hypertrophe Narbe darstellt.

6. Verfahren nach einem der Ansprüche 1 bis 3, worin der Zustand jedweden wie folgt darstellt: Sklerodermie; systemische Sklerose, CREST-Syndrom; tuberöse Sklerose mit Hautflecken; familiäres kutanes Kollagenom; metabolische und immunologische Störungen der Haut; eosinophile Fasziitis; diskoider Lupus erythematodes; Dermatomyositis; gemischte Bindcgewebserkrankung; arzneimittelinduzierte Hautfibrose; orale submuköse Fibrose; Diät- und Umwelt-induzierte Fibrose; pulmonale/kardiale Fibrose; Leberfibrose/Leberzirrhose; Nierenfibrose; arzneimittelinduzierte Fibrose; fibrose des zentralen und peripheren Nervensystems; Fibrose des Gefäßsystems; Fibrose des männlichen und weibliche Urogenitalsystems; und gynäkologische Fibrose.

7. Verfahren nach einem der Ansprüche 1 bis 3, worin der Zustand jedweden wie folgt darstellt: Zirrhose der Leber, Leberfibrose, Glomerulonephritis, pulmonale Fibrose, zystische Fibrose, Sklerodermie, Myokardfibrose, Fibrose nach einem Myokardinfarkt, und Fibrose des Zentralnervensystems nach einem Schlaganfall oder neurogenerativen Erkrankungen.

8. Verfahren nach einem der vorangehenden Ansprüche, worin die mitochondriale DNA aus Blut- oder Gewebeprohen isoliert wird, die einem Individuum entnommen worden sind und dann zum Nachweis der Anwesenheit eines genetischen Polymorphismus oder einer Mutation, der/die mit der Entwicklung des Zustands verknüpft ist, untersucht werden.

9. Verfahren nach einem der vorangehenden Ansprüche, worin die mitochondriale DNA amplifitziert wird, bevor sie zum Nachweis der Abwesenheit eines genetischen Polymorphismus oder einer Mutation untersucht wird.

10. Verfahren nach Anspruch 9, worin die mitochondriale DNA unter Verwendung der Polymerasekettenreaktion (PCR) amplifiziert wird.

11. Verfahren nach Anspruch 10, worin ein PCR-Primerpaar von SEQ ID NO: 9 und 10 oder ein Primerpaar von SEQ ID NO: 11 und 12 zum Amplifizieren der mitochondrialen DNA verwendet wird.

12. Verfahren nach einem der vorangehenden Ansprüche, worin das mitochondriale Genom mittels Restriktionsverdau und Größenanalyse untersucht wird.

13. Kit zur Verwendung im Verfahren nach einem der Ansprüche 10 bis 12, umfassend:
A) PCR-Primer zum Amplifizieren genetischer Polymorphismen oder Mutationen im mitochondrialen 16srRNA-Gen, die mit einem Zustand verknüpft sind, der durch eine ungünstige Fibrose oder Vernarbung **gekennzeichnet** ist;
B) Kontroll-DNA-Proben von bekanntem Genotyp für jeden Polymorphismus oder jede Mutation; und
C) eine Datenkarte, die die Verknüpfung zwischen einem bestimmten Polymorphismus und einem Zustand darlegt, dcr mindestens teilweise durch eine ungünstige Fibrose oder Vernarbung **gekennzeichnet** ist.

14. Kit nach Anspruch 13, worin die PCR-Primer nach Anspruch 11 definiert sind.

15. Kit nach Anspruch 13 oder 14, worin das Kit weiter ein geeignetes Restriktionsenzym zum Bilden von DNA-Fragmenten umfasst.

## Revendications

1. Procédé *in vitro* pour le diagnostic ou la détection d'une prédisposition à une affection au moins partiellement **caractérisée par** une fibrose ou une cicatrisation inappropriée, le procédé consistant à examiner le gène de l'ARNr 16S du génome mitochondrial d'un sujet intéressant pour détecter la présence d'un polymorphisme génétique ou d'une mutation lié(e) au développement de l'affection.

2. Procédé selon la revendication 1 dans lequel une région autour de la position 2839 du génome mitochondrial est examinée pour détecter la présence d'un polymorphisme génétique ou d'une mutation.

3. Procédé selon la revendication 2 dans lequel une insertion d'un nucléotide thymidine (T) après le nucléotide guanosine (G) (G2839G/T) à la position 2839 du génome mitochondrial est examinée.

4. Procédé selon l'une quelconque des revendications 1-3 dans lequel l'affection est la maladie de Dupuytren.

5. Procédé selon l'une quelconque des revendications 1-3 dans lequel l'affection est une chéloïde cicatricielle ou une cicatrice hypertrophique.

6. Procédé selon l'une quelconque des revendications 1-3 dans lequel l'affection est une affection quelconque parmi la sclérodermie; la sclérodermie généralisée; le syndrome CREST; la sclérose tubéreuse de Bourneville avec taches cutanées; le collagénome cutané, forme familiale; les troubles métaboliques et immunologiques de la peau; la fasciite à éosinophiles, le lupus érythémateux discoïde; la dermatomyosite; la collagénose mixte; la fibrose cutanée induite par les médicaments; la fibrose sous-muqueuse orale; la fibrose induite à la suite d'expositions alimentaires et environnementales; la fibrose pulmonaire/cardiaque; la cirrhose/fibrose hépatique; la fibrose rénale; la fibrose induite par les médicaments; la fibrose du système nerveux central et périphérique; la fibrose de l'appareil vasculaire; la fibrose de l'appareil urogénital mâle et femelle; et la fibrose gynécologique.

7. Procédé selon l'une quelconque des revendications 1-3 dans lequel l'affection est une affection quelconque parmi la cirrhose du foie, la fibrose hépatique, la glomérulonéphrite, la fibrose pulmonaire, la fibrose kystique, la sclérodermie, la fibrose myocardique, la fibrose qui suit un infarctus du myocarde, et la fibrose du système nerveux central à la suite d'un accident cérébrovasculaire ou de troubles neurodégénératifs.

8. Procédé selon l'une quelconque des revendications précédentes dans lequel l'ADN mitochondrial est isolé à partir d'échantillons sanguins ou tissulaires prélevés sur le sujet, puis examiné pour détecter la présence d'un polymorphisme génétique ou d'une mutation lié(e) au développement de l'affection.

9. Procédé selon l'une quelconque des revendications précédentes dans lequel l'ADN mitochondrial est amplifié avant d'être examiné pour détecter la présence d'un polymorphisme génétique ou d'une mutation.

10. Procédé selon la revendication 9 dans lequel l'ADN mitochondrial est amplifié en utilisant la réaction en chaîne de la polymérase (PCR).

11. Procédé selon la revendication 10 dans lequel une paire d'amorces de PCR de SEQ ID NO.9 et 10 ou une paire d'amorces de SEQ ID ND. 11 et 12 sont utilisées pour amplifier l'ADN mitochondrial.

12. Procédé selon l'une quelconque des revendications précédentes dans lequel le génome mitochondrial est examiné par digestion de restriction et analyse de la taille des fragments.

13. Trousse, à utiliser dans le procédé selon l'une quelconque des revendications 10-12, comprenant:
A) des amorces de PCR pour l'amplification de polymorphismes génétiques ou de mutations dans le gène de l'ARNr 16 S mitochondrial qui sont lié(e)s à une affection **caractérisée par** une fibrose ou une cicatrisation inappropriée;
B) des échantillons d'ADN témoins de génotype connu pour chaque polymorphisme ou mutation; et
C) une carte de données indiquant le lien entre un polymorphisme particulier et une affection au moines partiellement **caractérisée par** une fibrose ou une cicatrisation inappropriée.

14. Trousse selon la revendication 13 dans laquelle les amorces de PCR sont telles que définies dans la revendication 11.

15. Trousse selon les revendications 13 ou 14 où la trousse comprend en outre une enzyme de restriction appropriée pour la génération de fragments d'ADN.
